# EUROPEAN PATENT SPECIFICATION

(11) **EP 3 393 527 B1**
(45) Date of publication and mention of the grant of the patent: **28.08.2019**
(21) Application number: 16840310.3
(22) Date of filing: 22.12.2016
(51) Int. Cl.: A61K 49/00, A61K 49/18, A61P 35/00, A61K 31/337, A61K 31/704, A61K 9/00, A61K 9/51, A61K 9/107

(54) **FLUORESCENT CYPATE CONJUGATE OF HYALURONIC ACID OR SALT THEREOF, HYDROPHOBIZED CONJUGATE, METHODS OF PREPARATION AND USE THEREOF**
FLUORESZENTES CYPATKONJUGAT AUS HYALURONSÄURE ODER EINEM SALZ DAVON, HYDROPHOBIERTES KONJUGAT, VERFAHREN ZUR ZUBEREITUNG UND VERWENDUNG DAVON
CONJUGUÉ FLUORESCENT DE CYPATE ET D'ACIDE HYALURONIQUE OU D'UN SEL DE CELUI-CI, CONJUGUÉ HYDROPHOBISÉ, PROCÉDÉS DE PRÉPARATION ET D'UTILISATION ASSOCIÉS

(30) Priority: 23.12.2015 CZ 20150936
(43) Date of publication of application: 31.10.2018
(73) Proprietor: Contipro a.s., 56102 Dolni Dobrouc (CZ)
(72) Inventor: ACHBERGEROVA, Eva, 75142 Merovice nad Hanou (CZ); SMEJKALOVA, Daniela, 56206 Usti nad Orlici (CZ); HUERTA-ANGELES, Gloria, 56002 Ceska Trebova (CZ); SOUCEK, Karel, 66603 Malhostovice (CZ); HERMANNOVA, Martina, 56401 Zamberk (CZ); VELEBNY, Vladimir, 56401 Zamberk (CZ)
(74) Representative: Dvorakova, Martina
(86) International application number: PCT/CZ2016/050048
(87) International publication number: WO 2017/108015

(56) References cited:
- WO-A1-2014/082609
- US-A1- 2009 028 788
- KOJI MIKI ET AL: "Near-Infrared Dye-Conjugated Amphiphilic Hyaluronic Acid Derivatives as a Dual Contrast Agent for In Vivo Optical and Photoacoustic Tumor Imaging", BIOMACROMOLECULES, vol. 16, no. 1, 12 January 2015 (2015-01-12), pages 219-227, XP055366080, ISSN: 1525-7797, DOI: 10.1021/bm501438e cited in the application
- DATABASE WPI Week 201381 Thomson Scientific, London, GB; AN 2013-M47637 XP002769418, & KR 2013 0085294 A (UNIV IND & ACADEMIC COOP IN CHUNGNAM NAT) 29 July 2013 (2013-07-29)
- EENSCHOOTEN C ET AL: "Preparation and structural characterisation of novel and versatile amphiphilic octenyl succinic anhydride-modified hyaluronic acid derivatives", CARBOHYDRATE POLYMERS, APPLIED SCIENCE PUBLISHERS, LTD. BARKING, GB, vol. 79, no. 3, 11 February 2010 (2010-02-11), pages 597-605, XP026806649, ISSN: 0144-8617 [retrieved on 2009-09-19] cited in the application

## Description

Fluorescent conjugate of hyaluronic acid or salt thereof, hydrophobized conjugate, methods of preparation and use thereof.

### Field of the Invention

The present invention relates to a fluorescent ester conjugate of hyaluronic acid or a salt thereof containing *in vivo* diagnosable heptamethine indocyanine dye (Cypate) of the formula III or 1-[3-(2-carboxyethyl)-1,1-dimethyl-5,9b-dihydrobenzo[e]indol-3-ium-2-yl(chloride)]-octa-1,3,5,7-tetraenyl]-1,1-dimethyl-2H-benzo[e]indol-3-yl]propanoic acid. Further it is described a hydrophobized conjugate, methods of preparation thereof and an use for *in vivo* imaging applications and a treatment of neoplasms.

### Background of the invention

### Hyaluronic acid

Hyaluronic acid or a salt thereof (HA) is a linear polysaccharide belonging to the group of important glucosamino glycanes. In the terms of the structure, it is a biopolymer formed with repeated disaccharide units (*β*-(1→4) glycosidic bond), consisting of D-glucuronic acid and *N-*acetyl-D-glucosamine mutually linked with a (*β*-(1→3) glycosidic bond (see formula 1 below).

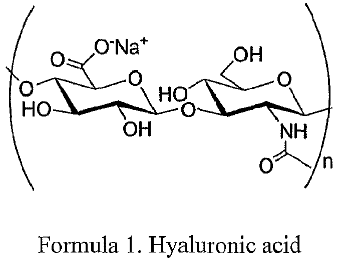

Hyaluronic acid occurs in the physiological environment in the form of sodium salt as a very hydrophilic and highly hydrated biopolymer (Schanté C. E; et al., Carbohydr. Polym. 2011, 85 (3), 469-489). HA plays an important role in the structure and organization of the extracellular matrix and also forms suitable environment for cells, their proliferation, differentiation and mobility (D'Este M.; et al., Carbohydr. Polym. 2014, 108, 239-246; Schanté, C. E.; et al., F. Carbohydr. Polym. 2011, 85 (3), 469-489). HA is further contained in vertebrates in all body organs and the extracellular matrix of soft connective tissues (Eenschooten, C.; et al., Carbohydr. Polym. 2010, 79 (3), 597-605).

This, in the water soluble polysaccharide is relatively easily subjected to the chemical modification and it appears as a biomolecule very suitable for a conjugation with other compounds, including fluorescent agents. An advantage of HA is also the existence of several cellular receptors thereof, which can be used for specific aiming of derivatives, for example, into tumor tissues (Garg H. G.; et al., Chemistry and biology of hyaluronan, 1st ed.; Elsevier: Netherlands, 2004).

### In vivo diagnostis

Recently, one of the suitable methods for noninvasive diagnostics, beside nuclear imaging technics, the roentgen radiography and the computer tomography, is the optical imaging through fluorescence. Fluorescence appears as a promising method for *in vivo* diagnostics, especially thanks to its relatively high sensitivity, specificity and image gaining in the real time (Ye, Y.; et al., Bioconjugate Chem. 2008, 19 (1), 225-234). Among advantages of the fluorescent imaging are also relatively low cost, feasibility, non-invasiveness and safety in comparison to the ionizing radiation. This technic is very perspective for the detection, diagnostics and prevention in the tumor therapy, but also as a complement method for gaining complex information in clinic applications in the imaging using the positron emission tomography (PET), SPECT ("single-photonemission computed tomography") or MRI. ("magnetic resonance imaging"), (Ye, Y.; et al., Theranostics 2011, 1, 102-106).

In the application of the non-invasive diagnostic methods is the penetration of irradiation through tissues highly dependent on absorption properties and the refractive index (Frangioni, J. V. Curr. Opin. Chem. Biol. 2003, 7 (5), 626-634). The absorption and emission wavelength in the area 650 - 900 nm, that is radiation in the near infrared area (NIR), are considered as optimal. In comparison with shorter wavelengths of visible spectra, the radiation of these wavelengths penetrates deeper and at the same time no absorption by the endogenous fluorophores and rise of undesired autofluorescence occurs (Kobayashi, H.; et al., Chem. Rev. 2010, 110, 2620-2640, Luo, S. et al.; Biomaterials 2011, 32 (29), 7127-7138).

### NIR fluorescent agents

Recently, cyanine fluorescent dyes, derivatives of squarine, phtalocyanines, porphyrines and also some agents derived from boron-dipyrromethen (BODIPY) (Luo, S.; et al., Biomaterials 2011, 32 (29), 7127-7138) belong among important exogenous contras agents.

Among very frequent agents used for imaging optical methods belong cyanine dyes. Structurally, they relate most to two heterocyclic structures, where one of these heterocycles carries positively charged nitrogen atom and they are further linked through the polymethine bridge, shown in the general formula of cyanine fluorescent agent, where

X-(CH=CH)*ₙ*-CH=Y

X and Y are heterocyclic nitrogen structures and n = 1 - 3. If *n* = 1, dyes count among tricyanine, *n* = 2 pentacyanine and *n* = 3 heptacyanine compounds.

Generally, the length of the polymethine bridge determines fluorescent properties of the given derivative and with every accruing n occurs the rise of the absorption and emission wavelengths of the compounds by about 100 nm. The typical wavelengths for the trimethine dyes are about 500 nm, for pentamethine about 600 nm and heptamethine derivatives eventually rich wavelengths of the near infrared area. Bathochromic and hypsochromic shift of wavelengths is further partially influenced by the type of the heterocyclic structure that also determines absorption and radiance of the fluorescent dye (Hermanson, G. T. Bioconjugate Techniques, 2. ed.; Elsevier: United States of America, 2008).

### Conjugates of NIR fluorescent agents with various molecules

In the past, it was possible to use NIR fluorescent agents alone only to nonspecific imaging, e.g. blood circuit and its purification (Frangioni, J. V. Curr. Opin. Chem. Biol. 2003, 7 (5), 626-634). Polymethine cyanine dyes tend to an aggregate in the aqueous environment, which results in the loss of their fluorescence and it is therefore disadvantageous in *in vivo* diagnostics (US6641798 B2). The said fluorescent agents are further characterized by very the short halftime of the degradation in the circuit system (150-180 s), that limits accumulation of the dye in the examined target, for example, in the tumor tissue and by that decreases also *in vivo* contrast (Hill T. K. et. al., Bioconjug Chem. 2015,; 26(2): 294-303). Simple and general approach how to increase the accumulation of the contrast agents in target places and tissues, or ensure the better solubility in the aqueous environment, is their conjugation with the suitable ligand from the group of peptides, proteins, saccharides etc. (Frangioni, J. V. Curr. Opin. Chem. Biol. 2003, 7 (5), 626-634). The conjugation of the contrast agents to the carrier polymer or the nanoparticle is mostly performed through a linker. The linker should be used to ensure fluorescent (to prevent quenching) and/or degradable conditions of system (Frangioni, J. V. Curr. Opin. Chem. Biol. 2003, 7 (5), 626-634). The disadvantage of this solution is that the reaction has more steps and it is very difficult, and derivatization of the entering agents is often used, which is disadvantageous in the term of the purification of the products and/or the isolation of the reaction intermediates is necessary, which is again disadvantageous. In the case of nanoparticle systems, the linker length has to be chosen so, that no fluorescence quenching of the contrast agent occurs and the contrast agent is *in vivo* detectable (i.e. the linker must not be too short) (US20110104070).

Patent document US6641798 posts the demands to the general structure of the low-molecular conjugates of the bioactive molecules (for example peptides, proteins, antibodies, saccharides) with the cyanine fluorescent agents, prepared to increase the detection and the therapy of the tumor. A serious disadvantage of used derivatives in in vivo application is the short stability of fluorescence (c. 45 min) at the imaging.

In literature there were also described conjugates of NIR fluorescent agent Cypate with the several different ligands (amino saccharides, peptides, polysaccharides) in term of the contrast agents for *in vivo* diagnostics. The preparation of NIR fluorescent agent Cypate itself is described in document WO2002032285, the improved synthesis was further published in the publication Ye, Y.; et al., Bioconjugate Chem. 2005, 16, 51-61. For *in vivo* studies prepared Yunpeng Ye.; *et al.* (Ye, Y.; et al., J. Am. Chem. Soc. 2004, 126, 7740-7741), polyvalent probes, where one or more monosaccharide units of D-(+)-glucosamine were bound on the carboxyl group of through the amide bond. Given NIR fluorescent agent formed the core of the dendrimeric formation and at the same time it served as a chromophore for nanoparticles, the biodistribution thereof was investigated using non-invasive optical methods *in vivo* and then *ex vivo.* One disadvantage of this solution, where no polymer was used for the conjugation, is insolubility of the system in the aqueous solutions in the absence of organic solvent.

Another type of known nanoparticle for tumor imaging is a dually targeted polymer micelle on the basis of succinyl chitosan with a covalently bond Cypate using the amide bond, further with methionine and folic acid (Chen, H.; et al., Polym. Chem. 2014, 5, 4734-4746). A disadvantage of this solution is using of chitosan as a carrier polysaccharide, as chitosan is a body-foreign agent. Another disadvantage is very limited solubility of the native chitosan in the physiological conditions, the nature chitosan must be therefore always modified, because otherwise it would not be possible to use chitosan in the intravenous application. Furthermore, the modified chitosan is usually limitedly soluble in the physiological conditions. The modification can cause changes in the biodegradability and biocompatibility of chitosan depending on the desired application (Balan, V.; et al., European Polymer Journal 2014, 53, 171-188; Dumitriu, S. Polymeric Biomaterials, 2nd ed.; Marcel Dekker, Inc.: United States of America, 2002). Chitosan conjugated with Pluronic F68 and with the cyanine dye (Cy5.5) was used in tracking the accumulation in the tumor mouse model (W. II Choi.; et al., Nanomedicine: Nanotechnology, Biology and Medicine 2015, 11, 359-368). Without targeting of the carrier system with herceptin was the tumor tracking imaging *in vivo* possible, however, after 12 hours the NIR fluorescence intensity did not continue to increase and it can be therefore supposed that this system does not enable the long term tracking of the tumor disease *in vivo* without the necessity of further carrier addition. The similar disadvantage of the short imaging time (maximum of 1-2 days after the application) is known also in the other polymer and non-polymer conjugates with NIR dyes (D. Kokuryo; et al., Journal of Controlled Release 2013, 178, 125, Tan X.; et al., Biomaterials 2012, 33, 2230-2239).

In literature, Cypate was further described as a part of multifunctional imaging probe, when on one carboxyl group of the said fluorescent agent was bound a chelating component of cation metals through the amide bond with the purpose of forming the optical-nuclear imaging system. Cypate was further conjugated to the cyclic RGD peptide with the purpose of targeting specific cells (Ye, Y.; et al., Bioconjugate Chem. 2008, 19, 225-234). The disadvantage of this system is that its fluorescent conditions change in the presence of metal cations and in some cases (presence of Fe³⁺ or Cu²⁺) can lead up to the complete quenching of fluorescence (Ye, Y.; et al., Bioconjugate Chem. 2008, 19, 225-234).

### Conjugates of NIR fluorescent agents with hyaluronic acid.

In publications Choi K. (Choi, K. Y.; et al., J. Mater. Chem. 2009, 19 (24), 4102-4107 and Choi, K. Y.; et al., Biomaterials 2010, 31 (1), 106-114), there was described a hydrophobized derivative of hyaluronic acid with 5β-cholic acid. However it was found that that the action of 5β-cholic acid leads to supporting of carcinogenesis of the intestine tumors in their early stages. (Baijal, K. P.; et al., Canadian Journal of Physiology and Pharmacology, 1998, 76(12), 1095-1102). Further, NIR fluorescent agent Cy5.5 was conjugated on the carboxyl group of hyaluronic acid, using ethyl-3(3-dimethylaminopropyl)-carbodiimide/hydroxy-benzotriazol. (EDC/HOBt) and linker dihydrazide of adipic acid. A disadvantage is not only multistep synthesis, but also used activator EDC/HOBt, that can cause undesirable intramolecular netting of hyaluronan and with that corresponding decrease of solubility of the final product (Huerta-Angeles, G.; et al., Carbohydr. Polym. 2014, 111 (13), 883-891). The derivative of hyaluronic acid conjugated with Cy5.5 through the amide bond was further used for the surface treatment of superparamagnetic nanoparticles iron oxides (SPION) with the purpose of forming multimodal probes for combination of *in vivo* MRI/optical detection of activity of hyaluronidase (Lee, D.; et al., Macromol. Res. 2011, 19 (8), 861-867). With respect to *in vivo* diagnostics through the optical method, the fluorescent agent Cy5.5 from the group of pentamethine derivatives is not the ideal choice. In contrast to heptamethine derivatives, they do not reach such high absorption and emission wavelengths, that are for *in vivo* non-invasive diagnostics recommended, that is in using wavelengths typical for pentamethine derivatives can create the undesirable autofluorescence of endogenous fluorophores.

In the case of heptamethine derivatives of cyanine is known the conjugate of hyaluronic acid with commercially available NIR fluorescent IR-783, modified four-step synthesis resulting in IR-783-S-Ph-COOH, where this derivative was consequently bound through the amide bond to hyaluronic acid. The derivative was prepared to study deeper understanding of pharmacokinetic of HA *in vivo,* its degradation and role in the physiological and pathological conditions (Wang, W.; Cameron, A. G.; Shi, K. Molecules 2012, 17, 1520-1534). Unfortunately, the very complicated synthesis is not transformable into the industrial scale either in terms of economic or in terms of very low yield (Wang, W.; Cameron, A. G.; Shi, K. Molecules 2012, 17, 1520-1534).

Among other representatives of this type of fluorescent agents belongs indocyanine green (ICG). HA conjugated with indocyanine green and polyethylene glycol (PEG) makes aggregated particles in the aqueous environment, with the possibility of *in vivo* dual imaging of the tumor using optical methods and the photoacoustic detection (Mild, K.; et al., Biomacromolecules 2015, 16, 219-227). The large disadvantage of using indocyanine green *in vivo* is hepatobiliary toxicity and fast removal of this dye by liver (G. R. Cherrick, et al. J. Clinical Investigation, 1960, 39, 592-600). The disadvantage of derivatives of HA with NIR fluorescent agents, when it comes to their conjugation onto the carboxyl group of hyaluronic acid and that in modification of the carboxyl function of HA, results in the neutralization of the natural negative charge of biopolymer, further can influence the solubility of HA in the physiological environment and not lastly also disturbs the recognition of HA by cell receptors (Mero, A.; et al., Carbohydr. Polym. 2010, 79 (3), 597-605).

### Summary of the Invention

The said disadvantages of the prior art are overcome by a fluorescent conjugate of hyaluronic acid or a salt thereof of the general formula I 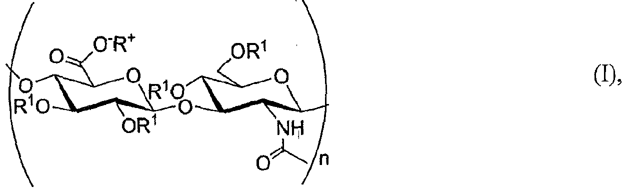
wherein R⁺ is H⁺ or a physiologically acceptable salt selected from the group containing Na⁺, K⁺, Mg²⁺ or Ca²⁺,
R¹ is -H or the cypate residue of the formula II, where ∼ is a place of covalent bond of a cypate residue of the formula II 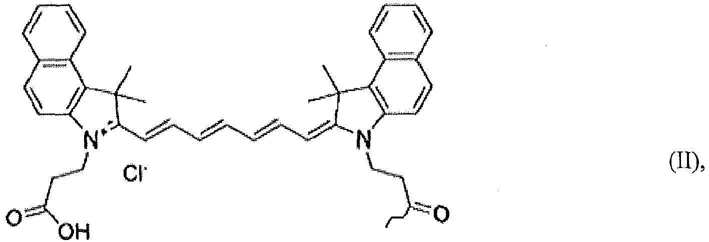
one R¹ being the cypate residue of the formula II in at least one repeated unit providing that if there is one R¹ cypate residue of the formula II in the unit, then the others R¹ in the unit are H,
and where n is an integer in the range of 2 to 625.

According to a preferred embodiment of the invention the cypate residue of the formula II is substituted at the position 6 of glucosamine part of the fluorescent conjugate of hyaluronic acid or the salt thereof of the general formula I.

Cypate I is bound to a hydroxyl group of hyaluronic acid (see Scheme 1 and 2, below) after the activation, which is advantageous especially in terms of maintaining the biological properties of hyaluronic acid and also of its solubility in the physiological environment. The solubility of the conjugate of the present invention is 1 to 3 mg per 100 µl of physiological solution. The fluorescent conjugate of the invention is excited and absorbs the light in the area from 570 nm to 790 nm and emits the light in the area from 680 to 850 nm, preferably at 850 nm.

The degree of substitution of the cypate residue of the formula II bound in the conjugate of hyaluronic acid or the salt thereof of the general formula I is from 0.1 to 2%, preferably 1.0%. The low degree of substitution of the cypate residue in the conjugate of the present invention is preferred, since it enables to the image distribution of the conjugate *in vivo,* without the structure of HA to be significantly modified.

The preparation of the conjugate itself lies in the synthesis of the fluorescent agent: Cypate, further in the activation of the carboxyl group of the fluorescent agent and following esterification of hyaluronic acid.

The activation of the carboxyl group of Cypate I of the formula III is performed using *N,N'-*carbonyl diimidalzole in the aprotic polar solvent (see Scheme 1, below) preferably selected from the group containing dimethyl sulfoxide (DMSO), dimethyl formamide (DMF), formamide, or acetonitrile, more preferably DMSO. This activation is very effective even under the mild reaction conditions, wherein the reaction of the carboxyl group with *N,N'*-carbonyl diimidazole (CDI) results in the reactive intermediate mono-imidazolide (Cypate II of formula IV), where the reaction is driven by the release of carbon dioxide and imidazole (see Scheme 1, below). The activation reaction runs for 10 minutes to 24 hours, preferably 0.5 to 2 hours. The reaction temperature can be in the range from 20 °C to 60 °C, preferably in the range from 22 °C to 25 °C.

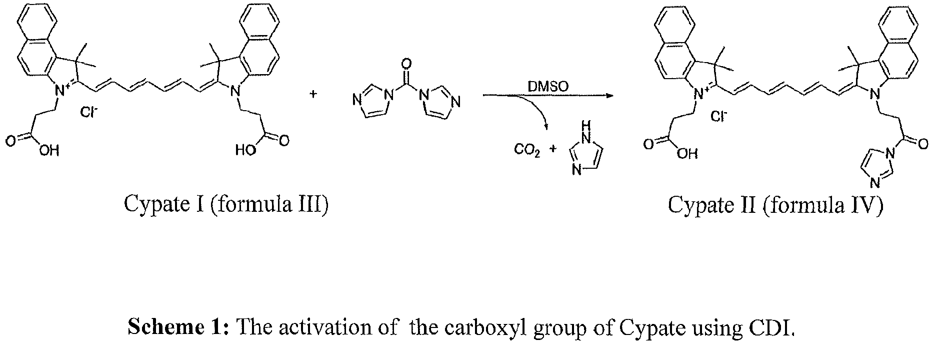

In the esterification of hyaluronic acid itself, shown in Scheme 2 (R⁺ is, as is defined above), is then imidazole eliminated from Cypate II from Scheme 1, and the ester bond between at least one hydroxyl group of hyaluronic acid and Cypate of formula IV is formed.

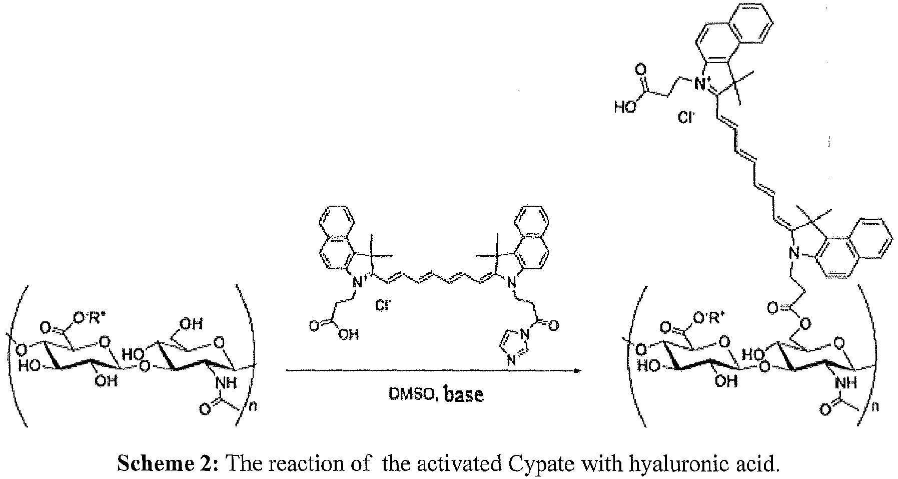

For the preparation of the conjugate, it is preferably used an acid form of hyaluronic acid or other organic salt for example tetrabutyl ammonium (TBA) (**Scheme 2**), that is used for the solubilization of hyaluronic acid in organic solvent (DMSO). Suitable molecule mass of the acid form or the other organic salt of hyaluronic acid for the given reaction is in the range from 5,000 - 250,000 g/mol, preferably (10,000 - 32,000 g/mol). Different molecule mass of HA of HA salt is not an obstacle for the reaction. The esterification of hydroxyl groups of HA in the aprotic polar solvent is further carried out by the addition of the fluorescent agent with the activated carboxyl group through CDI (N,N'-carbonyl diimidazole). The reaction runs at the presence of an organic base generated in situ in a form of imidazole or a added organic base selected for example from the group containing DABCO (1,4-diazabicyclo[2.2.2]octane), N,N,N',N'-tetramethyl-1,6-hexanediamine, N-methyl morpholine, imidazole, triethylamine (TEA) or N,N'-diisopropylethylamine (DIPEA), preferably imidazole generated in situ and the polar aprotic solvent, as is defined above. The reaction of the conjugate forming is performed at the temperature from 40 °C to 80 °C, preferably 40 °C to 60 °C, more preferably 60 °C for 12 to 48 hours, preferably 24 hours A closer study revealed that the degree of substitution of HA with the fluorescent agent is dependent on an equivalent amount of Cypate I and also it is positively influenced by the presence of an equivalent of the organic base, the preferred combination is 0.5 molar equivalent of Cypate I : 1 molar equivalent of HA : 0.5 molar equivalent of N,N'-carbonyl diimidazole : 0.5 to 3.5 molar equivalents of the organic base, more preferably 1 molar equivalent of the organic base. Thus, it applies that the molar ratio of Cypate I : hyaluronic acid or a salt thereof : N,N'-carbonyl diimidazole : organic base is 0.5 : 1 : 0.5 : 0.5 to 3.5 in the reaction mixture, preferably the molar ratio is 0.5 : 1 : 0.5 : 1. In the case of the organic base being generated *in situ* the molar ratio of cypate : hyaluronic acid or a salt thereof : N,N'-carbonyl diimidazole is 0.1 : 1 : 0.15 to 0.7 : 1 : 0.8, preferably 0.5 : 1 : 0.5.
The conjugate of hyaluronan with the heptamethine indocyanine fluorescent agent (or Cypate) of the general formula I, can be preferably further modified the forming of the hydrophobized fluorescent conjugate of the general formula I of the invention, where R⁺, R¹ and n are, as is defined above, applying at the same time, that at least in one repeated unit at least one R¹ is -C(=O)R², wherein R² is CₓH_{y} substituent, where x is an integer in the range of 5 to 17 and y is an integer in the range of 11 to 35, wherein it is a linear or branched, saturated or unsaturated C₆-C₁₈ aliphatic chain.

The degree of substitution of -C(=O)R² in the conjugate of hyaluronic acid or the salt thereof of the general formula I is from 1 to 70 %, preferably 5 to 12%. The hydrophobized fluorescent conjugate of the invention is excited and it absorbs the light in the range of wavelengths 570 nm to 790 nm and emits the light in the area of 680 to 850 nm, preferably at 850 nm.

The C₆ - C₁₈ acyl chain is the chain of fat acids that is linked through the ester bond to at least one hydroxyl group of HA. It binds preferably to the primary hydroxyl group (see Scheme 3), that is generally suitable for the esterification. The fat acid can have the short (SCFA), middle (MCFA), or long (LCFA) aliphatic chain and it can be essential or nonessential. The activation of the fat acid of the general formula V

R²COOH (V),

wherein R² is defined above,
is performed for example through the substituted or unsubstituted benzoyl chloride of the general formula VI 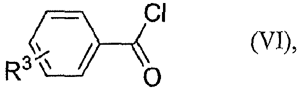 wherein R³ is one or more substituents selected from the group containing H, -NO₂, -COOH, halogenides, C₁-C₆ alkyl alkoxy, preferably H;
in the presence of the organic base selected from the group containing e.g. DABCO (1,4-diazabicyclo[2.2.2]octane), N,N,N',N'-tetramethyl-1,6-hexanediamine, N-methyl morpholine, triethylamine (TEA) or N,N'-diisopropylethylamine (DIPEA), preferably TEA. The example of the activation is shown in Scheme 3A. The reaction environment is made with the polar solvent selected from the group containing isopropyl alcohol (IPA), tetrahydrofuran (THF), preferably isopropyl alcohol, to form the reactive anhydride of the general formula VII 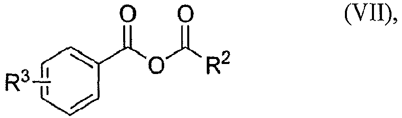 wherein
R² and R³ are, as is defined above, that esterifies the fluorescent conjugate of hyaluronic acid or a salt thereof of the general formula I of the present invention (for example shown in Scheme 3B).

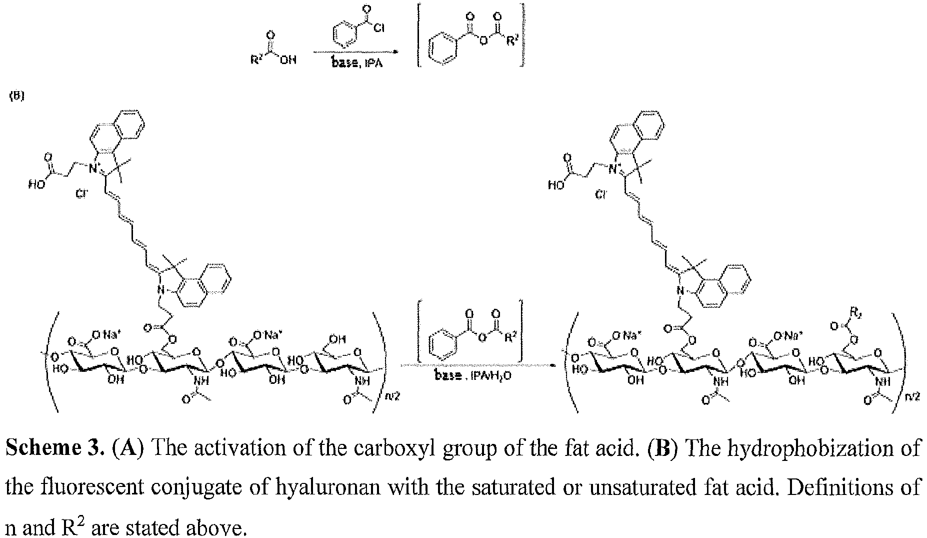

The esterification is performed with the activated carboxyl group of the fat acid in a mixture of water and the polar organic solvent miscible with water e.g. isopropyl alcohol (IPA), dimethyl sulfoxide (DMSO) or tetrahydrofuran (THF), preferably isopropyl alcohol. The esterification is performed in the mixture of water and the polar solvent miscible with water, wherein the water amount is in the range from 50 to 80 % v/v, preferably 50% v/v.

The reaction is also performed in the presence of the organic base preferably of amine selected from the group containing e.g. DABCO (1,4-diazabicyclo[2.2.2]octane), N,N,N',N'-tetramethyl-1,6-hexanediamine, N-methylmorpholine, imidazole, triethylamine (TEA) or N,N'-diisopropyl ethylamine (DIPEA), more preferably triethylamine. In the method of preparation of the hydrophobized conjugate of the present invention, the activation of the fat acid of the general formula V is performed for 0.5 to 24 hours, at the temperature in the range 0 °C to 60 °C, preferably 0.5 hours at the temperature 0 °C to 25 °C and the esterification of the fluorescent conjugate of hyaluronic acid or the salt thereof is performed for 0.5 to 2 hours, preferably 2 hours, at the room temperature, i.e.at the temperature in the range of 22 °C to 25 °C.

According to a preferred embodiment of the method of preparation of the hydrophobized conjugate of the present invention the amount of organic base corresponds to 2 to 6 molar equivalents, preferably to 4 molar equivalents per dimer of hyaluronic acid or the salt thereof. The amount of the substituted or unsubstituted benzoyl chloride corresponds to 0.2 to 2.0 molar equivalents, preferably 0.6 molar equivalents per dimer of hyaluronic acid or the salt thereof. The amount of the fat acid corresponds to 0.2 to 2.0 molar equivalents, preferably 0.6 molar equivalents per the dimer of hyaluronic acid or the salt thereof. The closer study found out, that the degree of substitution of the hydrophobized conjugate of hyaluronic acid or the salt thereof of the invention with the fat acid depends on the equivalent amount of the activated fat acid and also it is positively influenced by the presence of the organic base.

The hydrophobized conjugate of hyaluronan with the heptamethine indocyanine fluorescent agent (Cypate) of the general formula I of the present invention can be preferably used for the encapsulation (noncovalent bond) of nonpolar agents, preferably drugs or nanoparticles with hydrophobic surface. The hydrophobized conjugate is able to aggregate and form systems similar to polymer micelles with their behavior. Thus a composition is formed on the basis of the aggregated hydrophobized fluorescent conjugate of the present invention that contains aggregates of the hydrophobized fluorescent conjugates and at least one or more nonpolar agents, preferably drugs, more preferably cytostatics, most preferably doxorubicin or paclitaxel, and/or nanoparticles, preferably superparamagnetic nanoparticles (i.e. spions). Spions are preferably on the basis of iron oxides (Fe₂O₃, Fe₃O₄), where the amount of iron in the composition is 0.3 to 3 wt.% preferably 1 to 1.5 wt.%. The size of superparamagnetic nanoparticles is 4 to 6 nm, preferably 5 nm. In the preferred embodiment, the composition contains the aggregated hydrophobized fluorescent conjugate of the present invention, wherein R¹-C(=O)C₁₇H₃₃ and nanoparticles, that are preferably superparamagnetic nanoparticles on the basis of iron oxides (Fe₂O₃, Fe₃O₄). Such composition can further preferably contain cytostatic, preferably doxorubicin or paclitaxel.
According to another embodiment of the present invention, the composition contains 2 to 15 wt.% of nonpolar agents in respect to the mass content of the hydrophobized fluorescent conjugate of hyaluronic acid or the salt thereof of present invention, preferably 2 to 6 wt.%.

The compositions of the present invention can be used in medicinal applications for *in vivo* imaging of tumors or for treating tumors.

In comparison with so far the described processes of preparation of the conjugates of hyaluronic acid with the cyanine fluorescent agents, the stated method of preparation of the conjugate of present invention brings about several advantages. In contrast to the technics described in the publications, it relates to the direct synthesis without using any linker or previous modification of hyaluronic acid, or fluorescent agent. During the activation occurs the release of imidazole and CO₂ that are not toxic, and it can be easily removed from the reaction mixture. The release of imidazole can be further used in the preparation of the conjugate as in situ generated organic base and it need not be thus added as other organic base necessary for the reaction proceeding. The esterification of hyaluronic acid proceeds in the organic solvent e.g. in DMSO. The activator of cypate is CDI. CDI can be used for the conjugation of Cypate to HA without the necessity of isolation of the intermediate. The advantage in this case is that the selective modification of the primary hydroxyl of HA occurs even in the case of non-protected secondary hydroxyl groups of HA and no undesired side reaction like oxidation of HA in DMSO occurs (reaction Pfitzner-Moffatt).

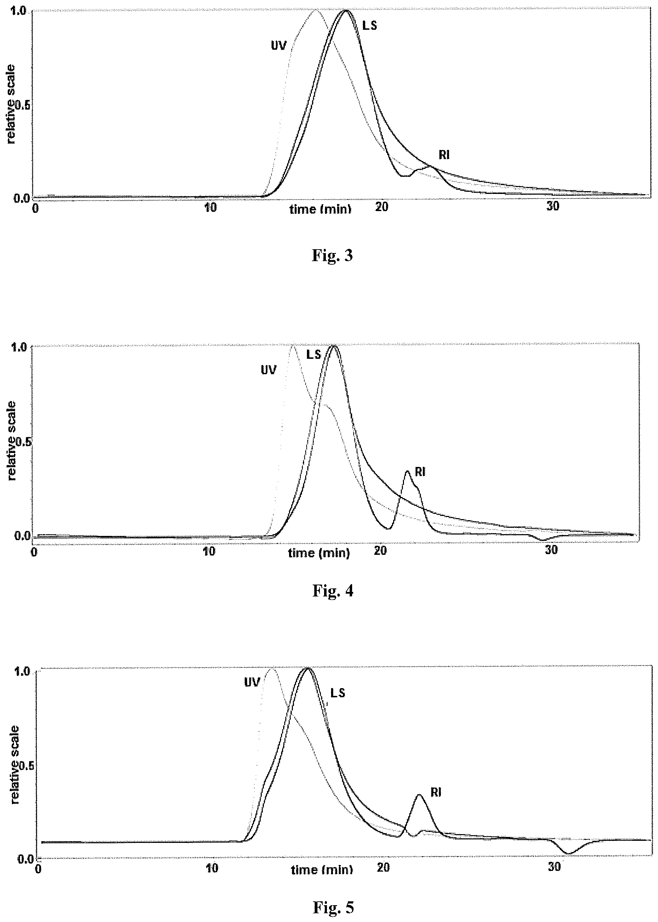

Although the structure of Cypate contains two functional carboxyl groups, surprisingly no netting of the fluorescent conjugate of HA occurs (see Fig. 3-5), which would lead to decrease of the final product solubility.

Similar ester derivatives of HA are not easy to obtain using other processes, or activators as are benzoyl chloride (BC) and 2,4,6-trichlorobenzoyl chloride (TBC) (WO2014082609). Neither ethyl chloroformiate can be used that can activate carboxyl group (WO 2012034544), as it leads to chromophore (cypate) degradation and loss of fluorescent properties. As another activator of carboxyl groups of hyaluronan in organic solvent is often used dicyclohexyl carbodiimide (DCC), its large disadvantage is, however, that it is indicated as strong allergen (Derm_Beruf. Umwelt. 1986; 34(4):110-1.) and it is highly toxic (Macrom. Rapid Commun. 2004, 25, 916-920).

The conjugate of hyaluronic acid with Cypate of general formula I and its hydrophobized conjugate of the present invention can be preferably excited in the area 570 nm to 790 nm and they emit at 680 to 850 nm, and thus suitable for the use in the medicinal applications for *in vivo* imaging of the conjugate distribution of the invention, preferably for *in vivo* imaging of organs selected from the group containing for example liver, skin; or imaging of tumors after the intravenous, intraperitoneal or subcutaneous administration.

These conjugates are able to penetrate after intravenous or intraperitoneal administration into tumor tissues (i.e. neoplasms), preferably into palpable tumors and/or into very small (non-palpable) tumors, and thus are suitable for the imaging to the diagnose disease, especially tumor disease. In comparison with the pentamethine compounds is heptamethine cyanine conjugate of hyaluronan advantageous in respect to fluorescent properties - especially of larger depth of the radiation penetration and further limitation of undesired autofluorescence. Diagnostics of tumor disease is preferably applicable for the tumor tissues selectively uptaking low-molecular hyaluronan (e.g. tissues with higher expression of CD44). The solubility of the hydrophobized conjugate of the present invention is 1 to 3 mg per 100 µl of the saline solution.

The hydrophobized conjugate of the present invention is in the term of the fluorescent properties very stable and after the intravenous administration it can be preferably imagined at least for 15 days without the necessity of the repeated administration of the conjugate. The conjugate of present invention is very easily concentrated even in small (palpably non-detectable) tumors. Another advantage of the hydrophobized conjugate of the present invention is the possibility of the noncovalent binding of the anticancer agent (cytostatic) and thus their usage for a construction of theranostics, i.e. carrier with diagnostic and therapeutic function at the same time. The main advantage of the hydrophobized conjugate of the present invention as a theranostic is the use of its ability to accumulate in tumor tissues (especially in breast tumors), long term imaging and application to treatment of tumor itself.

### Terms definition:

Cypate includes the structure of the general formula III (cypate I) or 1-[3-(2-carboxyethyl)-1,1-dimethyl-5,9b-dihydrobenzo[e]indol-3-ium-2-yl(chloride)]-octa-1,3,5,7-tetraenyl]-1,1-dimethyl-2H-benzo[e]indol-3-yl]propanoic acid, heptamethine indocyanine dye.

SS = degree of substitution = percentage amount of modified disaccharide units of hyaluronan per 100 disaccharide units of hyaluronan (100 % states, that per 100 of disaccharide units of hyaluronan was detected 100 modifying units)

The term "room temperature" refers to the range of temperatures in the room of 22 °C to 25 °C.

The equivalent (eqv.) refers to the dimer of hyaluronic acid, it refers to the molar equivalent if not stated otherwise.

The binding capacity is the amount of bound agent expressed in the percent by weight, if not stated otherwise.

The term "nonpolar agent" refers to a compound with symmetric distribution of charges. It refers to an agent that is soluble in organic solvents, especially in alcohols and insoluble in water.

### Description of figures in drawings.

Fig. 1: ¹H NMR (D₂O) conjugate HA-Cypate.
Fig. 2: DOSY NMR spectrum (D₂O) conjugate HA-Cypate.
Fig. 3: Chromatogram record of SEC-MALLS (HA-Cypate 14,000g/mol) conjugate HA-Cypate (Example 3).
Figr. 4: Chromatogram record of SEC-MALLS (HA-Cypate 14,000g/mol) conjugate HA-Cypate (Example 7).
Figr. 5: Chromatogram record of SEC-MALLS (HA-Cypate 58,000g/mol) conjugate HA-Cypate (Example 8).
Figr. 6: Chromatogram record of SEC-MALLS (HA-Cypate 72,000g/mol) conjugate HA-Cypate (Example 9).
Fig. 7: The emission spectrum of the fluorescence of conjugate HA-Cypate in the aqueous solution at the excitation 650, 660, 665 and 670 nm.
Fig. 8: The emission fluorescence of the conjugate in the aqueous solution at the excitation with laser λ= 632.8 nm without (left panel) and in a combination with the filter transmitting wavelengths over λ = 635 nm (right panel).
Fig. 9: *In vivo* fluorescent imaging: HA-Cypate applied subcutaneously. The place of application is indicated with letter S. Figures show the detection of emission using the different excitation and emission filters.
Fig. 10: *In vivo* fluorescent imaging in time after the intraperitoneal application of HA-Cypate.
Fig. 11: *In vivo* fluorescent imaging in time after the intravenous application of HA-Cypate-C18:1.
Fig. 12: *In vivo* fluorescent imaging in time after the intravenous application of HA-Cypate-C18:1 (mouse with tumor, tumor cells indicated with chemiluminescent luciferase).
Fig. 13: The evaluation of *in vivo* luminescence of tumor after the administration of (i) HA-Cypate-C18:1 (=HA cyp), (ii) HA-Cypate-C18:1+doxorubicin (=HA cyp dox), (iii) HA-Cypate-C18:1+doxorubicin+spion (=HA cyp dox+spions).
Fig. 14: The comparison of spleen and liver weight after the administration of (i) HA-Cypate-C18:1 (=HA cyp), (ii) HA-Cypate-C18:1+doxorubicin (=HA cyp dox), (iii) HA-Cypate-C18:1+doxorubicin+spion (=HA cyp dox+spions).
Fig. 15: *In vivo* fluorescent imaging in time after the intraperitoneal application of HA-Cypate-C18:1 (mouse with tumor, tumor cells indicated with luminescent luciferase).
Fig. 16: The mass spectrum of dimer HA-Cypate obtained from the enzymatic degradation of the conjugate HA-Cypate with the hyaluronan lyase.

### Examples of the Embodiments of the Present Invention

### Description of instrumentation

NMR spectra were recorded on BRUKER AVANCE 500 at frequency 500.13 MHz (¹H). For the processing of experimental data was used the software by Bruker TOPSPIN 1.2 or software SpinWorks 3.1. For the interpretation of the spectra from NMR analyses were used abbreviations: s (singlet), d (doublet), t (triplet), m (multiplet). For UV/Vis spectra measurement in the wavelength range 190 - 800 nm was used UV/Vis spectrophotometer Varian Cary 100. Fluorescent spectra were recorded on the apparatus PTI Quantamaster 400. ESI-MS analyses of Cypate were performed on the mass spectrophotometer with ionic trap amaZon X (BrukerDaltonics) equipped with the electrospray ionizing source and the quadrupole mass analyzer. Measurements were performed in the positive and negative mode. The structure of conjugate of HA-cypate was confirmed using LC-MS analysis of the conjugate after its enzymatic cleaving with hyaluronan lyase. The mixture of oligosaccharides was separated on column Kinetex 1.7 um F5 100A (Phenomenex) using gradient 0.1% HCOOH in H₂O and acetonitrile. The detection was performed on Synapt G2-Si in negative resolution mode with ionization with electrospray. Analysis of samples and molecule mass of initial hyaluronan was determined using the method SEC-MALLS (HPLC Alliance) with UV/VIS 2489 and the refractometry detector RID 2414 and the detector of light scattering mini DAWN TREOS. Data were processed using the software Astra Version 5.3.4.20 (Wyatt Technology EuropeGmbH). All *in vivo* imaging analyses were carried out on the apparatus IVIS Luminia XR Series III) on a laboratory mice of the strain Balb/c.

### Example 1. Synthesis of 3-(2-carboxyethyl)-1,2,2-trimethyl-1H-benzo[e]indolium-bromide

2.0 g (9.6 mmol) of 1,1,2-trimethyl-*1H*-benzindol and 2.2 g (14.3 mmol) 3-bromopropanoic acid were dissolved in 10 ml 1,2-dichlorobenzen and under the constant stirring warmed at 115 °C for 16 hours. The crude reaction mixture was cooled down to the room temperature and the resulting precipitate was washed with 1,2-dichloromethane (10 x 50 ml). The final product was separated using the filtration, dried under vacuum on the rotary evaporator (RE) and it was obtained in the form of light gray crystal powder (yield 2.2 g (64 %)).
¹H NMR (DMSO-*d₆*, 500 MHz): δ 8.38 (d, *J* = 8.35, 1Hₐᵣₒₘ), 8,29 (d, *J* = 9.00, 1Hₐᵣₒₘ), 8.23 (d, J= 8.35, 1Hₐᵣₒₘ), 8.18 (d, *J=* 9.00, 1Hₐᵣₒₘ), 7.80 - 7.71 (m, 2Hₐᵣₒₘ), 4.79 (t, *J=* 6.95, 2H, - CH₂-), 3.05 (t, *J* = 6.95, 2H, -CH₂-), 2.98 (s, 3H, -CH3), 1.76 (s, 6H,-CH3) ppm
ESI-MS: ⁺MS [M]⁺= 282

### Example 2. Preparation of Cypate

0.8 g (2.8 mmol) glutaconic dialdehyde dianiline hydrochloride was dissolved in 8 ml 1,2-dichloromethane and tempered to 0 - 5 °C. 521 µl (5.5 mmol) of the anhydride of acetic acid, 481 µl (2.8 mmol) and DIPEA in the small volume of 1,2-dichlormethan (0.5 ml) was added dropwise into the solution and the reaction mixture was left while cooling and stirring to react for 3 hours. In the meantime 2 g of (5.5 mmol) 3-(2-carboxyethyl)-1,2,2-trimethyl-1*H-*benzo[e]indolium-bromide prepared in Example 1 and 0.9 g (11.0 mmol) of sodium acetate in the mixture of solvents acetonitrile/water 95/5 of volume 15 ml were brought to reflux and the first reaction mixture was added dropwise. The reaction proceeded for 18 hours under stirring at reflux in dark. The crude reaction mixture was cooled down to the room temperature, washed with 400 ml ethyl-acetate and 400 ml 1M HCl, the product was filtrated and dried under the low pressure. The resulting product was obtained in the form of dark green crystal powder yielding 1.62 g (88 %).
¹H NMR (DMSO-d6, 500 MHz): δ 8.25 (d, *J* = 8.75, 2H), 8.07 - 7.97 (m, 6H), 7.82 (t, *J=* 12.65, 1H), 7.73 (d, *J* = 8.75, 2H), 7.69 - 7.62 (m, 2H), 7.54 - 7.48 (m, 2H), 6.60 (t, *J=* 12.65, 2H), 6.47 (d, *J=* 13.75, 2H), 4.43 (bt, 4H -CH₂-), 2.77 (t, *J=* 6.9, 4H -CH₂-), 1.92 (s, 12H -CH₃) ppm
ESI-MS: ⁺MS [M]⁺= 625; ⁻MS [M-2H]⁻= 623 m/z
UV/Vis: λ_{abs.max}= 782 nm (MeOH)

### Example 3: Preparation of conjugate HA-Cypate

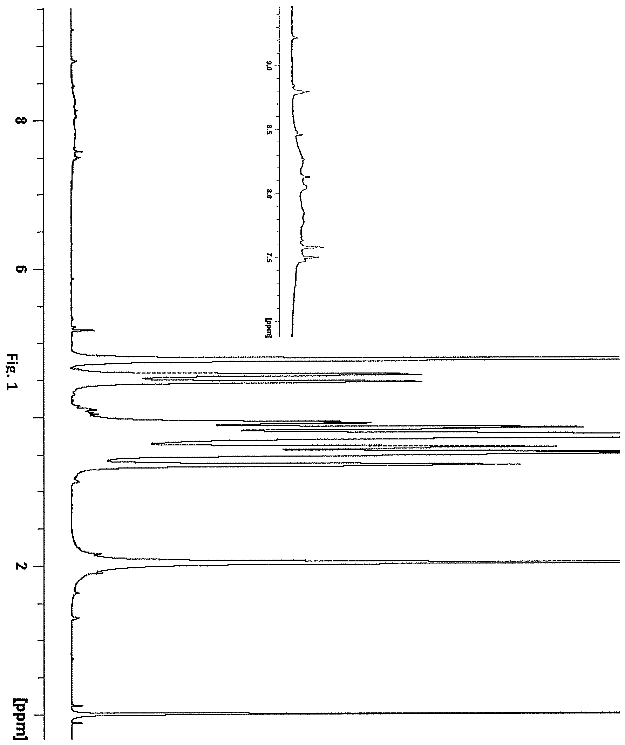

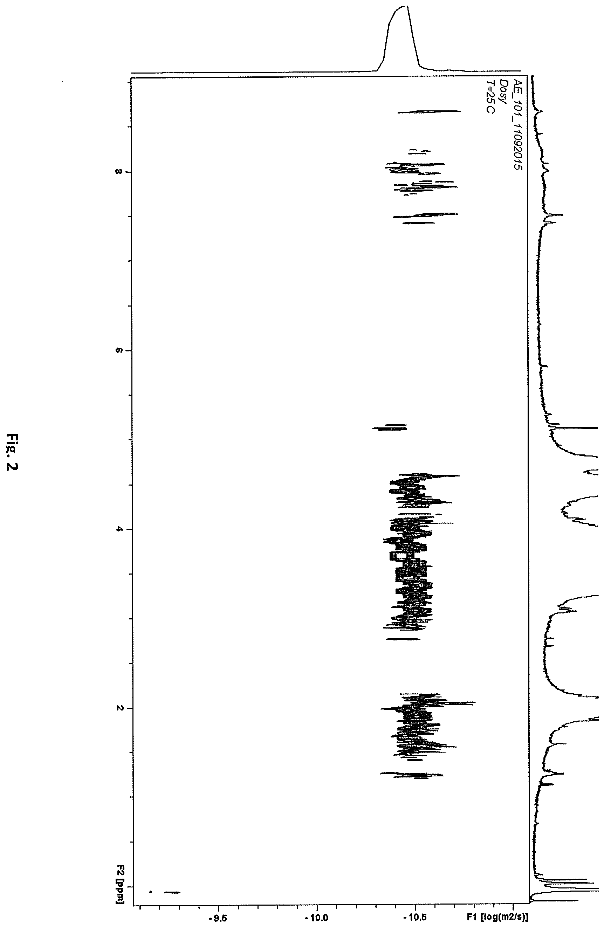

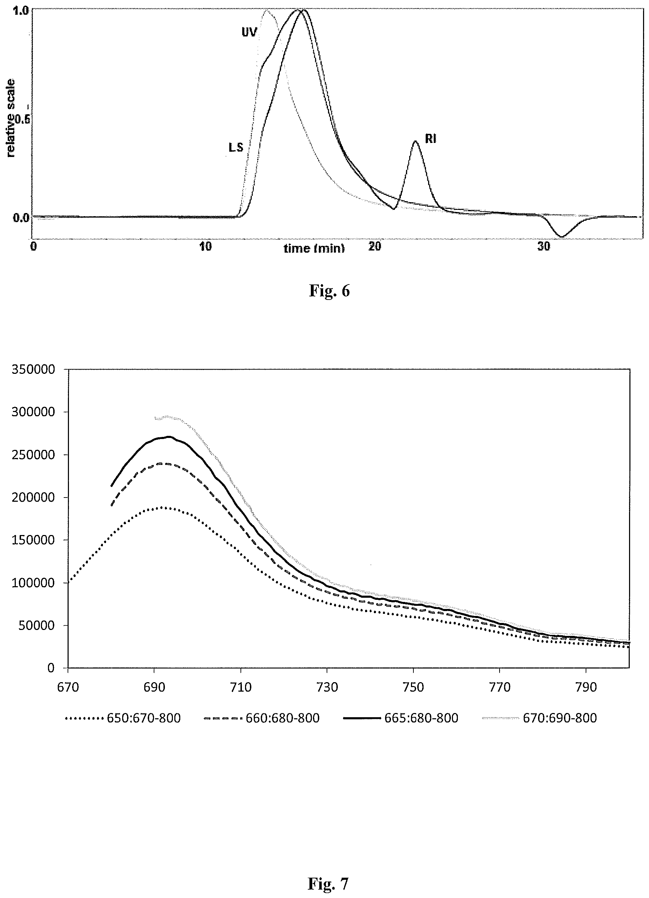

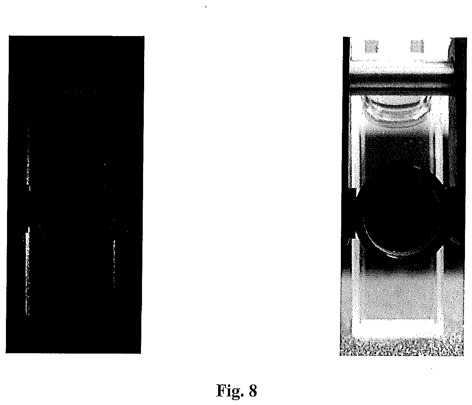

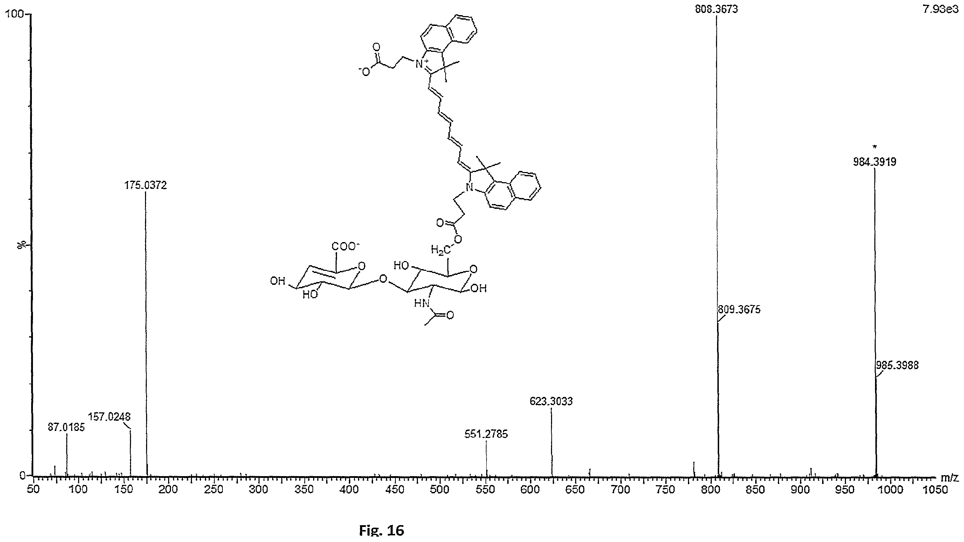

87 mg (0.13 mmol, 0.5 eqv.) Cypate from Example 2 was dissolved in 2 ml DMSO, 22 mg (0.13 mmol, 0.5 eqv.) *N,N'*-carbonyl diimidazole was added and under the constant stirring was let activate 2 hours at the room temperature. In the meantime 100 mg (0.27 mmol, 1 eqv.) acid form of hyaluronic acid M_{w} 14,000 g/mol was disolved in DMSO at 60 °C. Then 15 µl (0.13 mmol, 0.5 eqv.) *N*-methylmorfoline and the first reaction mixture without the previous isolation was added into the solution. The reaction proceeded under the constant stirring in dark at 60 °C 24 hours. The reaction was stopped by adding ten-fold of 100% isopropyl alcohol (AIPA) in respect to the initial volume of the reaction mixture and the saturated solution of NaCl, when precipitation of the desired product occurred. The crude product was purified with 5 x 100 ml AIPA, dissolved in 50 ml demineralized water and transferred into dialysis tube. The protonized form of HA was neutralized 1^{st} day in 0.5% solution of NaCl and 0.5% NaHCO₃, further dialyzed 2^{nd} and 3^{rd} day in the demineralized water. The tube content was frozen and lyofilized. The resulting product in the form of hyaluronan was obtained as green lyofilizate of mass 89 mg (87 %).
¹H NMR (D₂O) (Fig. 1): cypate: δ 8.80 (s, 2H), 8.50- 8.47 (m, 2H), 8.46 - 8.43 (m, 2H), 8.29 - 8.19 (m, 2H), 8.16 - 8.10 (m, 2H), 8.08 - 7.89 (m, 2H), 7.88 - 7.81 (m, 2H), 7.79 - 7.73 (m, 2H), 7.58 (s, 2H), 7.51 - 7.46 (m, 2H), 5.16, - 5.14 (m, 4H), 3.14 - 3.09 (m, 4H), 2.02 (12H, CH₃, overlaid with signals of HA), HA: δ 4.62 - 4.39 (m, 2H anomer), 4.01 - 3.28 (m, 10H skeletal), 2.02 (s, 3H, -CH₃) ppm
DOSY (D₂O): (Fig. 2)
SS = 1.5 % (determined from ¹H NMR)
SEC-MALLS-LS-UV/Vis-RI: (Fig. 3)
Fluorimeter: λ_{em.max}= 695 nm (at λ_{exc..} = 665 nm; H₂O); (Fig. 7)
Fluorescence of the conjugate excited with the laser wavelength λ= 632.8 nm (Fig. 8)
ESI-MS: [M-H]⁻= 984 m/z (Fig. 16, dimer detected after the enzymatic degradation of the conjugate with lyase)

### Example 4: Preparation of conjugate HA-Cypate

87 mg (0.13 mmol, 0.5 eqv.) Cypate from Example 2 was dissolved in 2 ml DMSO, 22 mg (0.13 mmol, 0.5 eqv.) *N,N'*-carbonyl diimidazole was added and under the constant stirring let to activate 30 min. at the room temperature. 100 mg (0.27 mmol, 1 eqv.) of the acid form of hyaluronic acid of M_{w} 14,000 g/mol in DMSO at 60 °C was added. The reaction proceeded under the constant stirring in dark at 60 °C for 24 hours. The reaction was stopped by addition of ten-fold volume of 100% isopropyl alcohol (AIPA) in respect to the initial volume of the reaction mixture and the saturated solution of NaCl, then the precipitation of the desired product occurred. The crude product was purified with 5 x 100 ml AIPA, dissolved in 50 ml demineralized water and transferred into the dialysis tube. The protonized form of HA was neutralized 1^{st} day in 0.5% solution NaCl and 0.5% NaHCO₃, further dialyzed 2^{nd} and 3^{rd} day in demineralized water. The tube content was frozen and lyofilized. The resulting product in the form of hyaluronan was obtained as green lyofilizate of mass 89 mg (87 %).
¹H NMR (D₂O) (Fig. 1): cypate: δ 8.80 (s, 2H), 8.50- 8.47 (m, 2H), 8.46 - 8.43 (m, 2H), 8.29 - 8.19 (m, 2H), 8.16 - 8.10 (m, 2H), 8.08 - 7.89 (m, 2H), 7.88 - 7.81 (m, 2H), 7.79 - 7.73 (m, 2H), 7.58 (s, 2H), 7.51 - 7.46 (m, 2H), 5.16, - 5.14 (m, 4H), 3.14 - 3.09 (m, 4H), 2.02 (12H, CH₃, overlaid with signals of HA), HA: δ 4.62 - 4.39 (m, 2H anomer), 4.01 - 3.28 (m, 10H skeletal), 2.02 (s, 3H, -CH₃) ppm
SS = 1 % (determined from ¹H NMR)
Fluorimeter: λ_{em.max}= 695 nm (at λ_{exc..} = 665 nm; H₂O)

### Example 5: Preparation of conjugate HA-Cypate

17 mg (0.03 mmol, 0.1 eqv.) Cypate from Example 2 was dissolved in 1 ml DMSO, 6 mg (0.04 mmol, 0.15 eqv.) *N,N'*-carbonyl diimidazole was added and under the constant stirring was let to activate 30 min. at the room temperature. 100 mg (0.27 mmol, 1 eqv.) of the acid form of hyaluronic acid of M_{w} 14,000 g/mol in DMSO was dissolved at 60 °C. The reaction proceeded under the constant stirring in dark at 60 °C 24 hours. The reaction was stopped with the addition of ten-fold volume of 100% isopropyl alcohol (AIPA) in respect to the initial reaction mixture volume and the saturated solution of NaCl, then the precipitation of the desired product occurred. The crude product was purified with 5 x 100 ml AIPA, dissolved in 50 ml demineralized water and transferred into the dialysis tube. The protonized form of HA was neutralized 1^{st} day in 0.5% solution of NaCl and 0.5% NaHCO₃, further dialyzed 2^{nd} and 3^{rd} day in the demineralized water. The tube content was frozen and lyofilized. The resulting product in the form of hyaluronan was obtained as green lyofilizate of mass 92 mg (90 %).
¹H NMR (D₂O): cypate: δ 8.80 (s, 2H), 8.50- 8.47 (m, 2H), 8.46 - 8.43 (m, 2H), 8.29 - 8.19 (m, 2H), 8.16 - 8.10 (m, 2H), 8.08 - 7.89 (m, 2H), 7.88 - 7.81 (m, 2H), 7.79 - 7.73 (m, 2H), 7.58 (s, 2H), 7.51 - 7.46 (m, 2H), 5.16, - 5.14 (m, 4H), 3.14 - 3.09 (m, 4H), 2.02 (12H, CH₃, overlaid with signals of HA), HA: δ 4.62 - 4.39 (m, 2H anomer), 4.01 - 3.28 (m, 10H skeletal), 2.02 (s, 3H, -CH₃) ppm
SS = 0.7 % (determined from ¹H NMR)
Fluorimeter: λ_{em.max} = 695 nm (at λ_{exc..} = 665 nm; H₂O)

### Example 6: Preparation of conjugate HA-Cypate

122 mg (0.19 mmol, 0.7 eqv.) Cypate from Example 2 was dissolved in 1 ml DMSO, 34 mg (0.21 mmol, 0.8 eqv.) *N,N'*-carbonyl diimidazole was added and under the constant stirring was let to activate 30 min. at the room temperature. 100 mg (0.27 mmol, 1 eqv.) of the acid form of hyaluronic acid of Mw 14,000 g/mol in DMSO was dissolved at 60 °C. The reaction proceeded under the constant stirring in dark at 60 °C 24 hours. The reaction was stopped with the addition of ten-fold volume of 100% isopropyl alcohol (AIPA) in respect to the initial reaction mixture volume and the saturated solution of NaCl, then the precipitation of the desired product occurred. The crude product was purified with 5 x 100 ml AIPA, dissolved in 50 ml demineralized water and transferred into the dialysis tube. The protonized form of HA was neutralized 1^{st} day in 0.5% solution of NaCl and 0.5% NaHCO₃, further dialyzed 2^{nd} and 3^{rd} day in the demineralized water. The tube content was frozen and then lyophilized. The resulting product was obtain in the form of hyaluronan as green lyofilizate of mass 85 mg (88 %).
¹H NMR (D₂O): cypate: δ 8.80 (s, 2H), 8.50- 8.47 (m, 2H), 8.46 - 8.43 (m, 2H), 8.29 - 8.19 (m, 2H), 8.16 - 8.10 (m, 2H), 8.08 - 7.89 (m, 2H), 7.88 - 7.81 (m, 2H), 7.79 - 7.73 (m, 2H), 7.58 (s, 2H), 7.51 - 7.46 (m, 2H), 5.16, - 5.14 (m, 4H), 3.14 - 3.09 (m, 4H), 2.02 (12H, CH₃, overlaid with signals of HA), HA: δ 4.62 - 4.39 (m, 2H anomer), 4.01 - 3.28 (m, 10H skeletal), 2.02 (s, 3H, -CH₃) ppm
SS = 1.3 % (determined from ¹H NMR)
Fluorimeter: λ_{em.max} = 695 nm (at λ_{exc..} = 665 nm; H₂O)

### Example 7: Preparation of conjugate HA-Cypate

87 mg (0.13 mmol, 0.5 eqv.) Cypate from Example 2 was dissolved in 2 ml DMSO, 22 mg (0.13 mmol, 0.5 eqv.) CDI was added and under the constant stirring was let to activate 2 h at the room temperature. 100 mg (0.27 mmol, 1 eqv.) the acid form of hyaluronic acid of Mw (14,000 g/mol) was dissolved in DMSO at 60 °C. Then was 138 µl (0.79 mmol, 3 eqv.) DIPEA and the first reaction mixture added to this solution. The reaction proceeded under the constant stirring in dark at 60 °C 24 hours.

The reaction was stopped by the addition of ten-fold volume of AIPA in respect to the initial volume of the reaction mixture and the saturated solution of NaCl, then occurred the precipitation of the desired product. The product was purified with 5 x 100 ml AIPA, dissolved in 50 ml demineralized water and transferred in the dialysis tube. The protonized form of HA was neutralized 1^{st} day in 0.5% solution of NaCl and 0.5% NaHCO₃, further dialyzed 2^{nd} and 3^{rd} day in demineralized water. The tube content was frozen and lyofilized. The resulting product in the form of hyaluronan was obtained as lyofilizate of mass 86 mg (84 %).
¹H NMR (D₂O): cypate: δ 8.81 (s, 2H), 8.50- 8.47 (m, 2H), 8.46 - 8.43 (m, 2H), 8.29 - 8.19 (m, 2H), 8.16 - 8.10 (m, 2H), 8.08 - 7.89 (m, 2H), 7.88 - 7.81 (m, 2H), 7.79 - 7.73 (m, 2H), 7.58 (s, 2H), 7.51 - 7.46 (m, 2H), 5.16, - 5.14 (m, 4H), 3.14 - 3.09 (m, 4H), 2.03 (12H, CH3, overlaid with signals of HA), HA: δ 4.62 - 4.38 (m, 2H anomer), 4.01 - 3.26 (m, 10H skeletal), 2.03 (s, 3H, -CH₃) ppm
SS = 1.5 % (determined from ¹H NMR)
SEC-MALLS-LS-UV/Vis-RI: (Figure 4)
Fluorimeter: λ_{em.max} = 695 nm (at λ_{exc.max} = 665 nm; H₂O)

### Example 8: Preparation of conjugate HA-Cypate

87 mg (0.13 mmol, 0.5 eqv.) Cypate from Example 2 was dissolved in 2 ml DMSO, 22 mg (0.13 mmol, 0.5 eqv.) CDI was added and under the constant stirring let activate for 2h at the room temperature. 100 mg (0.27 mmol, 1 eqv.) of the acid form of hyaluronic acid of Mw (5.8 x 10⁴ g/mol) in DMSO was dissolved at 60 °C. Into this solution was then added 15 µl (0.13 mmol, 0.5 eqv.) *N*-methyl morfolin and the first reaction mixture without the previous purification. The reaction proceeded under the constant stirring in dark at 60 °C 24 hours.

The reaction was quenched by the addition of ten-fold of AIPA in respect to the initial volume of the reaction mixture and saturated solution of NaCl, then the precipitation of the desired product occurred. The crude product was purified with 5 x 100 ml AIPA, dissolved in 50 ml demineralized water and transferred into the dialysis tube. The protonized form of HA was neutralized 1^{st} day in 0.5 % solution NaCl and 0.5% NaHCO₃, further dialyzed 2^{nd} and 3^{rd} day in demineralized water. The tube content was frozen and lyofilized. The resulting product in the form of hyaluronan was obtained as green lyofilizate of mass 95 mg (93 %).
¹H NMR (D₂O): cypate: δ 9.2 (m, 2H), 8.82- 8.77 (m, 2H), 8.46 - 8.43 (m, 2H), 8.29 - 8.19 (m, 2H), 8.16 - 8.10 (m, 2H), 8.08 - 7.89 (m, 2H), 7.89 - 7.81 (m, 2H), 7.79 - 7.73 (m, 2H), 7.58 (s, 2H), 7.51 - 7.46 (m, 2H), 5.16, - 5.14 (m, 4H), 3.14 - 3.09 (m, 4H), 2.03 (12H, CH₃, overlaid with signals of HA), HA: δ 4.64 - 4.38 (m, 2H anomer), 4.04 - 3.21 (m, 10H skeletal), 2.03 (s, 3H, -CH₃) ppm
[SS = 1.0 % (determined from ¹H NMR)
SEC-MALLS-LS-UV/Vis-RI: (Fig.5)
Fluorimeter: λ_{em.max}= 695 nm (at λ_{exc.} = 665 nm; H₂O))

### Example 9: Preparation of conjugate HA-Cypate

87 mg (0.13 mmol, 0.5 eqv.) Cypate from Example 2 was dissolved in 2 ml DMSO, 22 mg (0.13 mmol, 0.5 eqv.) *N,N'*-carbonyl diimidazole was added and with the constant stirring let activate for 2h at the room temperature. 100 mg (0.27 mmol, 1 eqv.) acid form of hyaluronic acid of Mw (7.2 x 10⁴ g/mol) in DMSO was dissolved at 60 °C. 15 µl (0.13 mmol, 0.5 eqv.) *N-*methyl morfoline and the first reaction mixture was then added into this solution. The reaction proceeded with the constant stirring in dark at 60 °C 24 hours.

The reaction was stopped by addition of ten-fold volume of AIPA in respect to the initial volume of the reaction mixture and the saturated solution of NaCl, then occurred the precipitation of the desired product. The crude product was purified with 5 x 100 ml 100% AIPA, dissolved in 50 ml demineralized water and transferred into the dialysis tube. The protonized form of HA was neutralized 1^{st} day in 0.5% solution NaCl and 0.5% NaHCO₃, further dialyzed 2^{nd} and 3^{rd} day in demineralized water. The tube content was frozen and lyofilized. The resulting product in the form of hyaluronan was obtained as lyofilizate of mass 96 mg (94 %).
¹H NMR (D₂O): cypate: δ 9.2 (m, 2H), 8.83- 8.5 (m, 2H), 8.45 - 8.43 (m, 2H), 8.30 - 8.18 (m, 2H), 8.16 - 7.99 (m, 2H), 7.99 - 7.89 (m, 2H), 7.89 - 7.81 (m, 2H), 7.79 - 7.73 (m, 2H), 7.58 (s, 2H), 7.51 - 7.46 (m, 2H), 5.16, - 5.14 (m, 4H), 3.14 - 3.09 (m, 4H), 2.03 (12H, CH3, overlaid with signals of HA), HA: δ 4.64 - 4.38 (m, 2H anomer), 4.04 - 3.21 (m, 10H skeletal), 2.03 (s, 3H, -CH₃) ppm
SS = 1.0 % (determined from ¹H NMR)
SEC-MALLS-LS-UV/Vis-RI: (Fig. 6)
Fluorimeter: λ_{em.max}= 695 nm (at λ_{exc.} = 665 nm; H₂O))

### Example 10: Preparation of conjugate HA-Cypate

87 mg (0.3 mmol, 0.5 eqv.) Cypate from Example 2 was dissolved in 2 ml DMSO, 22 mg (0.13 mmol, 0.5 eqv.) *N,N'*-carbonyl diimidazole was added and under the constant stirring let activate 2h at the room temperature. 100 mg (0.27 mmol, 1 eqv.) of the acid form of hyaluronic acid of Mw (2.5 x 10⁵ g/mol) was dissolved in DMSO at 40 °C. *N*-methyl morfolin and first reaction mixture was then added 15 µl (0.13 mmol, 0.5 eqv.) into this solution. The reaction proceeded under the constant stirring in dark at 40 °C 24 hours

The reaction was stopped by the addition of ten-fold volume of AIPA in respect to the initial volume of the reaction mixture and the saturated solution of NaCl, then the precipitation of desired product occurred. The crude product was purified with 5 x 100 ml AIPA, dissolved in 50 ml demineralized water and transferred into the dialysis tube. The protonized form of HA was neutralized 1^{st} day in 0.5% solution NaCl and 0.5% NaHCO₃, further dialyzed 2^{nd} and 3^{rd} day in demineralized water. The tube content was frozen and lyofilized. The resulting product in the form of hyaluronan was obtained as lyofilizate of mass 93 mg (92 %).
¹H NMR (D₂O): cypate: δ 9.2 (m, 2H), 8.83- 8.5 (m, 2H), 8.45 - 8.43 (m, 2H), 8.30 - 8.17 (m, 2H), 8.16 - 7.99 (m, 2H), 7.99 - 7.89 (m, 2H), 7.89 - 7.81 (m, 2H), 7.79 - 7.73 (m, 2H), 7.58 (s, 2H), 7.51 - 7.42 (m, 2H), 5.16, - 5.14 (m, 4H), 3.14 - 3.09 (m, 4H), 2.03 (12H, CH3, overlaid with signals of HA), HA: δ 4.62 - 4.38 (m, 2H anomer), 4.01 - 3.26 (m, 10H skeletal), 2.03 (s, 3H, -CH₃) ppm
SS = 0.5 % (determined from ¹H NMR)
Fluorimeter: λ_{em.max} = 695 nm (at λ_{exc.} = 665 nm; H₂O)

### Example 11: Esterification of HA-Cypate with hexanoic acid

300 mg (0.73 mmol, 1 eqv.) of the conjugate HA-Cypate prepared according to Example 3 was dissolved in 15 ml demi water and 13 ml AIPA, 382 µl (2.20 mmol, 3 eqv.) DIPEA and 4.5 mg (0.04 mmol, 0.05 eqv) DMAP were then added. 165 µl (1.32 mmol, 1.8 eqv.) hexanoic acid was dissolved in 2 ml AIPA, 255 µl (1.46 mmol, 2 eqv.) DIPEA 153 µl (1.32 mmol, 1.8 eqv.) benzoyl chloride was added and let react under the constant stirring for 30 minutes at 0 °C. After the period of time was everything quantitatively transferred to the first reaction mixture, then the esterification proceeded for 2 hours at the room temperature.

The reaction was quenched by addition of the high excess of AIPA and the saturated solution of NaCl, whereas the precipitation of product occurred. The crude product was washed with 4 x 200 ml AIPA, further dissolved in demineralized water and transferred into the dialysis tube. Dialysis proceeded for 24 hours against 0.5% NaHCO₃, 0.5% NaCl and further 8 hours in demineralized water. The product was obtained in the form of green lyofilizate yielding 230 mg (73%).
¹H NMR additional signals compared with example 3 (D₂O, 500 MHz): δ 2.4 (m, 2H, _{α}CH₂), 1.6 (m, 2H, _{β}CH₂), 1.31 (m, 4H, _{γ}CH₂), 0.8 (m, 3H, -CH₂-CH₃) ppm
SS (acylation) = 70 % (determined from ¹H NMR)
Fluorimeter: λ_{em.max} = 700 nm (at λ_{exc.} = 665 nm; H₂O)

### Example 12: Esterification of HA-Cypate with palmitic acid

300 mg (0.73 mmol, 1 eqv.) of the conjugate of HA-Cypate prepared according to Example 3 was dissolved in 15 ml demi water, and then was added 306 µl (2.20 mmol, 3 eqv.) TEA and 4.5 mg (0.04 mmol, 0.05 eqv.) DMAP were added. In the meantime 94 mg (0.37 mmol, 0.5 eqv.) of hexadecanoic acid dissolved in 3 ml THF, 153 µl (1.10 mmol, 1.5 eqv.) TEA, 43 µl (0.37 mmol, 0.5 eqv.) benzoyl chloride was added and let react under the constant stirring for 30 minutes at the room temperature. After the period of time was everything quantitatively transferred to the first reaction mixture, the esterification then proceeded for 2 hours at the room temperature.

The reaction was quenched by addition of the high excess of AIPA and saturated solution of NaCl, whereas the precipitation of the product occurred. The crude product was washed with 4 x 200 ml AIPA, further dissolved in demineralized water and transferred into the the dialysis tube. Dialysis proceeded for 24 hours against 0.5% NaHCO₃ and 0.5% NaCl and further for 48 hours in demineralized water. The product was obtained in the form of green lyofilizate yielding 189 mg (61%).
¹H NMR additional signals to example 3 (D₂O, 500 MHz): δ 2.76 - 2.66 (m, 2H, _{α}CH₂), 1.64 - 1.48 (m, 2H, _{β}CH₂), 1.31 - 1.08 (m, 24H, CH₂), 0.98 - 0.78 (m, 3H, -CH₂-CH₃) ppm
SS acylation = 7 % (determined from ¹H NMR)
Fluorimeter: λ_{em.max} = 710 nm (at λ_{exc.} = 685 nm; H₂O)

### Example 13: Esterification of HA-Cypate with palmitic acid

200 mg (0.49 mmol, 1 eqv.) of the conjugate of HA-Cypate prepared according to Example 3 was dissolved in 10 ml demi water, and then 136 µl (0.98 mmol, 2 eqv.) TEA and 3 mg (0.02 mmol, 0.04 eqv.) DMAP were added. 38 mg (0.16 mmol, 0.3 eqv.) of palmitic acid dissolved in 3 ml THF, 68 µl (0.48 mmol, 1 eqv.) TEA, 17 µl (0.16 mmol, 0.3 ekv.) benzoyl chloride was added and let react with the constant stirring for 30 minutes at the room temperature. After the period of time was everything quantitatively transferred to the first reaction mixture, the esterification then proceeded for 2 hours at the room temperature.

The eaction was quenched by the addition of high excess of AIPA and saturated solution of NaCl, whereas the precipitation of product occurred. The crude product was washed with 4 x 200 ml AIPA, further dissolved in demineralized water and transferred into the dialysis tube. Dialysis proceeded for 24 hours against 0.5% NaHCO₃ and 0.5% NaCl and further for 48 hours in demineralized water. The product was obtained in the form of green lyofilizate yielding 76 mg (37 %).
¹H NMR (D₂O, 500 MHz) additional signals to Example 3: δ 2.76 - 2.66 (m, 2H, _{α}CH₂), 1.64 -1.48 (m, 2H, _{β}CH₂), 1.31 - 1.08 (m, 24H, CH₂), 0.98 - 0.78 (m, 3H, -CH₂-CH₃) ppm
SS (acylation) = 5 % (determined from ¹H NMR)
Fluorimeter: λ_{em.max} = 710 nm (at λ_{exc.} = 685 nm; H₂O)

### Example 14: Esterification of HA-Cypate with palmitic acid

200 mg (0.49 mmol, 1 eqv.) of the conjugate HA-Cypate prepared according to Example 3 was dissolved in 10 ml demi water, and then 255 µl (1.47 mmol, 3 eqv.) DIPEA and 3 mg (0.02 mmol, 0.04 eqv.) DMAP were added. 38 mg (0.15 mmol, 0.3 eqv.) palmitic acid was dissolved in 3 ml THF, 85 µl (0.4-9 mmol, 1 eqv.) DIPEA, 17 µl (0.15 mmol, 0.3 eqv.) benzoyl chloride was added and let react under the constant stirring for 30 minutes at the room temperature. After the period of time was everything quantitatively transferred to the first reaction mixture, the esterification then proceeded for 2 hours at the room temperature.

The reaction was quenched by the addition of high excess of AIPA and the saturated solution of NaCl, whereas the precipitation of the product occurred. The crude product was washed with 4 x 200 ml AIPA, further dissolved in demineralized water and transferred into the dialysis tube. Dialysis proceeded for 24 h against 0.5% NaHCO₃ and 0.5% NaCl and further for 48 hours in demineralized water. The product was obtained in the form of green lyofilizate yielding 93 mg (45 %)..
¹H NMR (D₂O, 500 MHz) additional signals to Example 3: δ 2.76 - 2.66 (m, 2H, _{α}CH₂), 1.64 - 1.48 (m, 2H, _{β}CH₂), 1.31 -1.08 (m, 24H, CH₂), 0.98 - 0.78 (m, 3H, -CH₂-CH₃) ppm
SS (acylation) = 5 % (determined from ¹H NMR)
Fluorimeter: λ_{em.max} = 710 nm (at λ_{exc.} = 685 nm; H₂O)

### Example 15: Esterification of HA-Cypate with oleic acid

300 mg HA-Cypate (0.73 mmol, 1 eqv.) from Example 3 was dissolved in 15 ml demi water and 13 ml AIPA, 306 µl (2.20 mmol, 3 eqv.) TEA and 4.5 mg (0.04 mmol, 0.05 eqv.) DMAP were then added. 186 µl (0.59 mmol, 0.8 eqv.) cis-octadec-9-enoid acid dissolved in 2 ml AIPA, 306 µl (2.20 mmol, 3 eqv.) TEA, 68 µl (0.59 mmol, 0.8 eqv.) benzoyl chloride was added and everything was let react under the constant stirring for 30 minutes at the room temperature. After the period of time was everything quantitatively transferred to the first reaction mixture, esterification then proceeded for 2 hours at the room temperature.

The reaction was quenched by the addition of the high excess of AIPA and saturated solution of NaCl, whereas the precipitation of product occurred. The crude product was washed 4 x 200 ml AIPA, further dissolved in demineralized water and transferred into the dialysis tube. Dialysis proceeded for 24 hours against 0.5% NaHCO₃ and 0.5% NaCl and further 48 hours in demineralized water. The roduct was obtained in the form of green lyofilizate yielding 210 mg (66 %).
¹H NMR (D₂O, 500 MHz) additional signals to Example 3: δ 5.41 - 5.34 (m, 2H, CH=CH), 2.48 - 2.40 (m, 2H, -CH₂-CO-), 1.68 - 1.54 (m, 2H, -CH₂-CH₂,-CO-), 1.40 - 1.23 (m, 24 H, (-CH₂)₁₂), 0.91 - 0.68 (m, 3H,-CH₂-CH₃) ppm
SS (acylation) = 10 % (determined from ¹H NMR)
Fluorimeter: λ_{em.max}= 710 nm (at λ_{exc.} = 685 nm; H₂O)

### Example 16: Esterification of HA-Cypate with oleic acid

300 mg (0.73 mmol, 1 eqv.) of the conjugate of HA-Cypate prepared according to Example 3 was dissolved in 15 ml demi water and 13 ml AIPA, then 306 µl (2.20 mmol, 3 eqv.) TEA and 4.5 mg (0.04 mmol, 0.05 eqv.) DMAP were added. 116 µl (0.36 mmol, 0.5 eqv.) of cis-octadec-9-enoic acid dissolved in 2 ml AIPA, 153 µl (1.08 mmol, 1.5 eqv.) TEA, 43 µl (0.36 mmol, 0.5 eqv.) benzoyl chloride was added and let react under the constant stirring for 30 minutes at the room temperature. After the period of time everything was quantitatively transferred to the first reaction mixture, the esterification then proceeded for 2 hours at the room temperature.

The reaction was quenched with the addition of the high excess of AIPA and saturated solution of NaCl, whereas the precipitation of the product occurred. The crude product was washed with 4 x 200 ml AIPA, further dissolved in demineralized water and transferred into the dialysis tube. Dialysis proceeded for 24 hours against 0.5% NaHCO₃ and 0.5% NaCl and further for 48 hours in demineralized water. The product was obtained in the form of green lyofilisate yielding 205 mg (73 %).
¹H NMR (D₂O, 500 MHz) additional signals to Example 3: 5.41 - 5.34 (m, 2H, CH=CH), 2.48 - 2.40 (m, 2H, -CH₂-CO-), 1.68 - 1.54 (m, 2H, -CH₂-CH₂,-CO-), 1.40 - 1.23 (m, 24 H, (-CH₂)₁₂), 0.91 - 0.68 (m, 3H,-CH₂-CH₃) ppm
SS (acylation) = 8 % (determined from ¹H NMR)
Fluorimeter: λ_{em.max} = 710 nm (at λ_{exc.} = 685 nm; H₂O)

### Example 17: Esterification of HA-Cypate with oleic acid

300 mg (0.73 mmol, 1 eqv.) of the conjugate of HA-Cypate prepared according to Example 3 was dissolved in 15 ml demi water and 13 ml AIPA, 382 µl (2.20 mmol, 3 eqv.) DIPEA and 4.5 mg (0.04 mmol, 0.05 eqv.) DMAP were added. 139 µl (0.45 mmol, 0.6 eqv.) of cis-octadec-9-enoic acid dissolved in 2 ml AIPA, 229 µl (1.32 mmol, 1.8 eqv.) DIPEA, 51 µl (0.44 mmol, 0.6 eqv.) benzoyl chloride was added and let react under the constant stirring 30 minutes at 0 °C. After the given period was everything quantitatively transferred to the first reaction mixture, and the esterification proceeded for 2 hours at the room temperature.

The reaction was quenched by the addition of high excess of AIPA and saturated solution of NaCl, whereas the precipitation of the product occurred. The crude product was washed with 4 x 200 ml AIPA, further dissolved in demineralized water and transferred into the dialysis tube. Dialysis proceeded for 24 hours against 0,5% NaHCO₃ and 0,5% NaCl and further for 48 hrs in demineralized water. The product was obtained in the form of green lyofilizate yielding 204 mg (65 %).
¹H NMR (D₂O, 500 MHz) additional signals to Example 3: δ 5.41 - 5.34 (m, 2H, CH=CH), 2.48 - 2.40 (m, 2H, -CH₂-CO-), 1.68 - 1.54 (m, 2H, -CH₂-CH₂,-CO-), 1.40 - 1.23 (m, 24 H, (-CH₂)₁₂), 0.91 - 0.68 (m, 3H,-CH₂-CH₃) ppm
SS (acylation) = 12 % (determined from ¹H NMR)
Fluorimeter: λ_{em.max.} = 710 nm (at λ_{exc.} = 685 nm; H₂O)

### Example 18: Esterification of HA-Cypate with oleic acid

300 mg (0.73 mmol, 1 eqv.) of the conjugate HA-Cypate prepared according to Example 3 was dissolved in 15 ml demi water and 11 ml AIPA, then was 306 µl (2.20 mmol, 3 eqv.) TEA and 4.5 mg (0.04 mmol, 0.05 eqv.) DMAP added. 231 µl (0.73 mmol, 1 eqv.) of cis-octadec-9-enoic acid dissolved in 2 ml AIPA, 204 µl (1.46 mmol, 2 eqv.) TEA, 85 µl (0.73 mmol, 1 eqv.) benzoyl chloride were added and let react with the constant stirring for 30 minutes at the room temperature. After the given period of time everything was quantitatively transferred into the first reaction mixture, and the esterification proceeded for 2 hours at the room temperature.

The reaction was quenched by the addition of the high excess of AIPA and saturated solution of NaCl, whereas the precipitation of the product occurred. The crude product was washed with 4 x 200 ml AIPA, further dissolved in the demineralized water and transferred into dialysis tube. Dialysis proceeded for 24 hours against 0.5% NaHCO₃ and 0.5% NaCl and further for 48 hours in demineralized water. The product was obtained in the form of green lyofilizate yielding 226 mg (70 %).
¹H NMR (D₂O, 500 MHz) additional signals to Example 3: δ 5.41 - 5.34 (m, 2H, CH=CH), 2.48 - 2.40 (m, 2H, -CH₂-CO-), 1.68 - 1.54 (m, 2H, -CH₂-CH₂,-CO-), 1.40 - 1.23 (m, 24 H, (-CH₂)₁₂), 0.91 - 0.68 (m, 3H,-CH₂-CH₃) ppm
SS (acylation) = 12 % (determined from ¹H NMR)
Fluorimeter: λ_{em.max.} = 710 nm (at λ_{exc.} = 685 nm; H₂O)

### Example 19: Esterification of HA-Cypate with oleic acid

300 mg (0.73 mmol, 1 eqv.) of the conjugate HA-Cypate prepared according to Example 3 was dissolved in 15 ml demi water and 11 ml AIPA, then 375 µl (1.76 mmol, 2.4 eqv.) N,N,N',N'-tetramethyl-1,6-hexanediamine and 4.5 mg (0.04 mmol, 0.05 eqv.) DMAP was added. 139 µl (0.44 mmol, 0.6 eqv.) of cis-octadec-9-enoic acid dissolved in 2 ml AIPA, 375 µl (1.76 mmol, 2.4 eqv.) N,N,N',N'-tetramethyl-1,6-hexanediamine, 51 µl (0.44 mmol, 0.6 eqv.) benzoyl chloride were added and let react under the constant stirring for 30 minutes at the room temperature. After the given period everything was quantitatively transferred into the first reaction mixture, and the esterification proceeded for 2 hours at the room temperature.

The reaction was quenched by the addition of the high excess of AIPA and saturated solution of NaCl, whereas the precipitation of the product occurred. The crude product was washed with 4 x 200 ml AIPA, further dissolved in demineralized water and transferred into the dialysis tube. Dialysis proceeded for 24 hours against 0.5% NaHCO₃ and 0.5% NaCl and further for 48 hrs in demineralized water. The product was obtained in the form of green lyofilizate yielding 217 mg (70 %).
¹H NMR (D₂O, 500 MHz) additional signals to Example 3: δ 5.41 - 5.34 (m, 2H, CH=CH), 2.48 - 2.40 (m, 2H, -CH₂-CO-), 1.68 - 1.54 (m, 2H, -CH₂-CH₂,-CO-), 1.40 - 1.23 (m, 24 H, (-CH₂)₁₂), 0.91 - 0.68 (m, 3H,-CH₂-CH₃) ppm
SS (acylation) = 6 % (determined from ¹H NMR)
Fluorimeter: λ_{em.max.} = 710 nm (at λ_{exc.} = 685 nm; H₂O)

### Example 20: Esterification of HA-Cypate with oleic acid

300 mg (0.73 mmol, 1 eqv.) of the conjugate HA-Cypate prepared according to Example 3 was dissolved in 15 ml demi water and 11 ml AIPA, and then 241 µl (2.20 mmol, 3 eqv.) N-methyl morfoline and 4.5 mg (0.04 mmol, 0.05 eqv.) DMAP were added. 139 µl (0.44 mmol, 0.6 eqv.) cis-octadec-9-enoic acid dissolved in 2 ml AIPA, 193 µl (1.76 mmol, 2.4 eqv.) N-methyl morfoline, 51 µl (0.44 mmol, 0.6 eqv.) benzoyl chloride were added and let react under the constant stirring for 30 minutes at the room temperature. After the given period was everything quantitatively transferred into the first reaction mixture, and the esterification proceeded for 2 hours at the room temperature.

The reaction was quenched by the addition of the high excess of AIPA and saturated solution of NaCl, whereas the precipitation of the product occurred. The crude product was washed with 4 x 200 ml AIPA, further dissolved in demineralized water and transferred into the dialysis tube. Dialysis proceeded for 24 hours against 0.5% NaHCO₃ and 0.5% NaCl and further for 48 hrs in demineralized water. Product was obtained in the form of green lyofilizate yielding 264 mg (82 %).
¹H NMR (D₂O, 500 MHz) additional signals to Example 3: δ 5.41 - 5.34 (m, 2H, CH=CH), 2.48 - 2.40 (m, 2H, -CH₂-CO-), 1.68 - 1.54 (m, 2H, -CH₂-CH₂,-CO-), 1.40 - 1.23 (m, 24 H, (-CH₂)₁₂), 0.91 - 0.68 (m, 3H,-CH₂-CH₃) ppm
SS (acylation) = 12 % (determined from ¹H NMR)
Fluorimeter: λ_{em.max} = 710 nm (at λ_{exc.} = 685 nm; H₂O)

### Example 21: Esterification of HA-Cypate with oleic acid

300 mg (0.73 mmol, 1 eqv.) of the conjugate HA-Cypate prepared according to Example 3 was dissolved in 15 ml demi water and 11 ml AIPA, and then 246 mg (2.20 mmol, 3 eqv.) DABCO and 4.5 mg (0.04 mmol, 0.05 eqv.) DMAP were added. 139 µl (0.44 mmol, 0.6 eqv.) cis-octadec-9-enoic acid dissolved in 2 ml AIPA, 148 mg (1.32 mmol, 1.8 eqv.) DABCO, 51 µl (0.44 mmol, 0.6 eqv.) benzoyl chloride were added and let react with the constant stirring for 30 minutes at the room temperature. After the given period everything was quantitatively transferred into the first reaction mixture, and the esterification proceeded for 2 hours at the room temperature.
The reaction was quenched by the addition of the high excess of AIPA and saturated solution of NaCl, whereas the precipitation of product occurred. The crude product was washed with 4 x 200 ml AIPA, further dissolved in demineralized water and transferred into the dialysis tube. Dialysis proceeded for 24 hours against 0.5% NaHCO₃ and 0.5% NaCl and further 48 hours in demineralized water. The product was obtained in the form of green lyofilizate yielding 183 mg (60 %).
¹H NMR (D₂O, 500 MHz) additional signals to Example 3: δ 5.41 - 5.34 (m, 2H, CH=CH), 2.48 - 2.40 (m, 2H, -CH₂-CO-), 1.68 - 1.54 (m, 2H, -CH₂-CH₂,-CO-), 1.40 - 1.23 (m, 24 H, (-CH₂)₁₂), 0.91 - 0.68 (m, 3H,-CH₂-CH₃) ppm
SS (acylation) = 3 % (determined from ¹H NMR)
Fluorimeter: λ_{em.max} = 710 nm (at λ_{exc.} = 685 nm; H₂O)

### Example 22: Loading of hydrophobized HA-Cypate with nonpolar compound

150 mg of the acylated conjugate of HA-cypate prepared according to Example 16 was 2 hours dissolved in 15 ml demi water under the constant stirring on the magnetic stirrer. Then was gradually added 10 mg paclitaxel in 2 ml chloroform and the resulting mixture was evaporated (RE) to dry and then hydrated with demi water (15 ml). Unbound paclitaxel was removed with the filtration through 1.0 µm glass filter and the resulting product was lyofilized.
The amount of unbound paclitaxel (HPLC determination): 4.2% (wt.)

### Example 23: Loading of hydrophobized HA-Cypate with nonpolar compound

150 mg of the acylated conjugate HA-cypate prepared according to Example 18 was 2 hours dissolved in 15 ml demi water with the constant stirring on the magnetic stirrer. Then 15 mg doxorubicin was gradually added in 2 ml chloroform and the resulting mixture was first sonicated (pulse sonication cca 15 min, 200 W, amplitude 65%, cycle 0.5 s) until reaching of the homogenous mixture and then evaporated (RE) to dry and then hydrated with demi water (15 ml). Unbound doxorubicin was removed with filtration through 1.0 µm glass filter and the resulting product was lyofilized.

The amount of unbound doxorubicin (HPLC determination): 7.5% (wt.)

### Example 24: Loading of hydrophobized HA-Cypate with nonpolar compound

150 mg of the acylated conjugate HA-cypate prepared according to Example 18 was 2 hours dissolved in 15 ml demineralized water with the constant stirring on the magnetic stirrer. Then 2 mg of spions on the basis of iron (Fe₂O₃, Fe₃O₄) (5 mm) and 20 mg doxorubicin was gradually added in 5 ml chloroform and the resulting mixture was first sonicated (pulse sonication, cca 15 min, 200 W, amplitude 65%, cycle 0.5s) until reaching homogenous mixture and then evaporated (RE) to dry and then hydrated with demi water (15 ml). Unbound doxorubicin was removed with filtration through 1.0 µm glass filter and the resulting product was lyofilized.

The amount of unbound doxorubicin (HPLC determination): 6.5% (wt.), unbound spions: 2% (wt.)

### Example 25: In vivo experiment - fluorescence of conjugate HA-Cypate

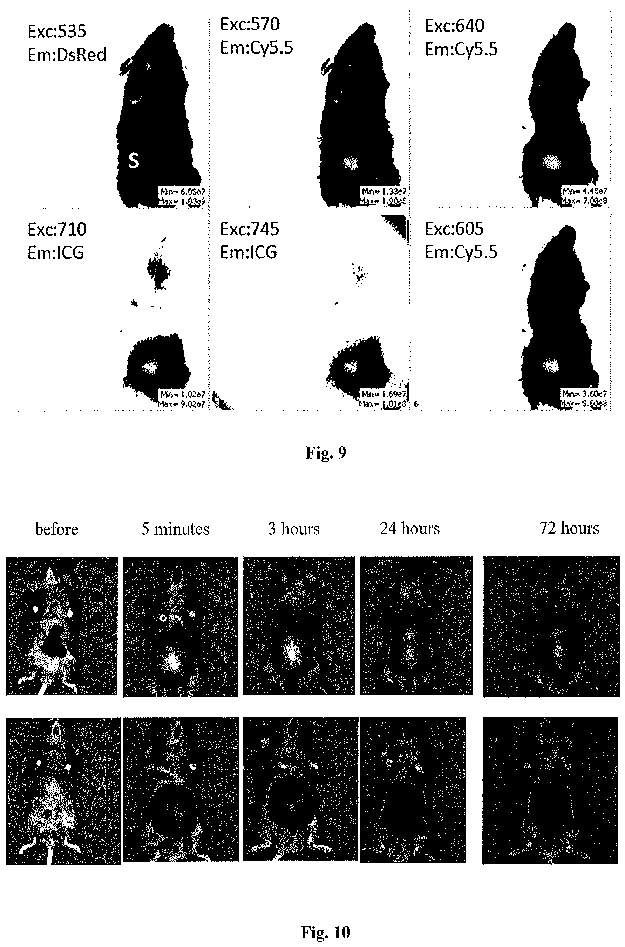

For *in vivo* experiments was HA-Cypate, prepared according to Example 3, dissolved in the saline solution (c = 3.8 mg derivative in 100 µl solution) and then sterilized by filtration (0.22 µm). 50 µl (Fig. 9) and/or intraperitoneally 150 µl (Fig. 10) sterile solution of the given derivative was subcutaneously applied to the laboratory mouse of the type Balb/c in narcosis. Fluorescence was detected using the combination of different excitation wavelengths and emission filters.

Fig. 9 a 10 show the sufficient intensity of fluorescence of the derivative after the subcutaneous and intraperitoneal application for *in vivo* imaging. The imaging can be performed using the various excitation wavelengths (530-745 nm), and filters for the emission (ICG, Cy5.5). The filter DsRed cannot be used. From Fig. 10 the stability of the derivative as fluorophore after intraperitoneal administration is further apparent.

### Example 26: In vivo experiment - fluorescence of conjugate HA-Cypate-C18:1

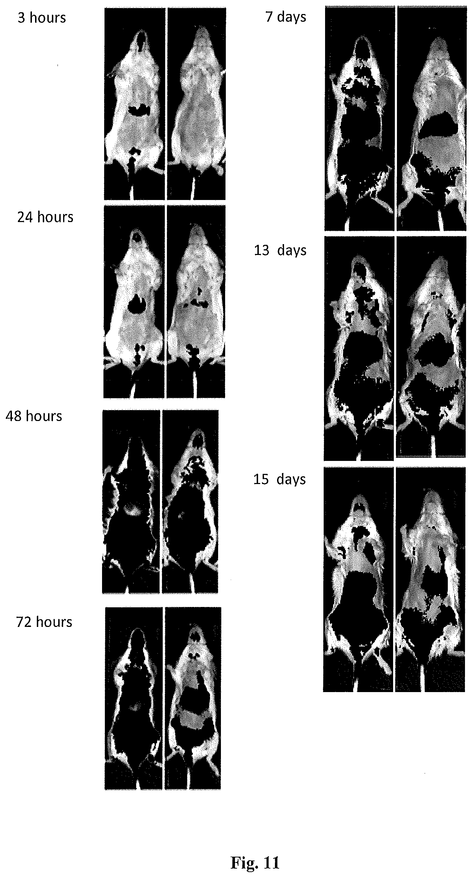

HA-Cypate-C18:1 (SS =10 %) prepared according to Example 15 was dissolved in phosphate buffer (c = 3.6 mg of the conjugate in 100 µl solution and sterilized by filtration (0.22 µm). 100 µl of such prepared solution was applied intravenously to two model mice Balb/c (in narcosis) and observed its fluorescence *in vivo* for the period of 7 days (Fig. 11).

Fig. 11 show that HA-Cypate-C18:1 is distributed after i.v. administration in the healthy mouse especially into liver. Fluorescence of the conjugate is sufficient for *in vivo* imaging, fluorescence of the conjugate is further very stable, after one administration can be imaging performed for 2 weeks.

### Example 27: In vivo experiment-fluorescence of conjugate HA-Cypate-C18:1

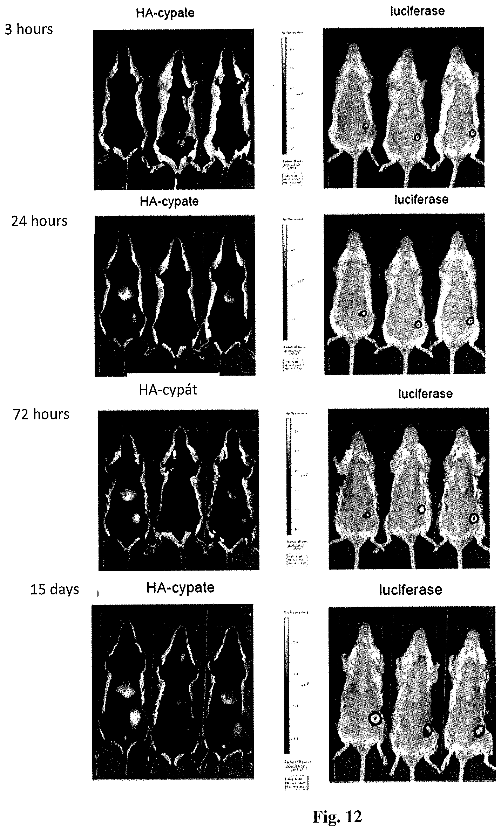

HA-Cypate-C18:1 (SS =10 %) prepared according to Example 15 was dissolved in the saline solution (c = 1.8 mg of the conjugate in 100 µl solution) and sterilized by the filtration (0.22 µm). 100 µl of such prepared solution was applied intravenously to 3 model mice Balb/c (in narcosis) with non-palpable breast tumor (orthotopic administration 4T1 luc cells, displayable through chemiluminescence -detection of luciferase activity after i.p. injection of luciferin) and observable its fluorescence *in vivo* for period 72 hours (Fig. 12).

Fig. 12 shows that HA-Cypate-C18:1 is distributed after i.v. administration into liver and further after 24 hours into very small (non-palpable) tumor. In tumor, there is the accumulation of HA-Cypate-C18:1 growing with time and the presence of the conjugate in tumor is very significant even after 15 days after the administration of the conjugate. Fluorescence of the conjugate *in vivo* is thus very stable and the conjugate can be used to imaging of very small tumors and further to tumor observation in time. In left panel of the figure is the control image of tumor with the luminescent imaging (the detection of luciferase activity).

### Example 28: In vivo experiment - fluorescence of conjugate HA-Cypate-C18:1

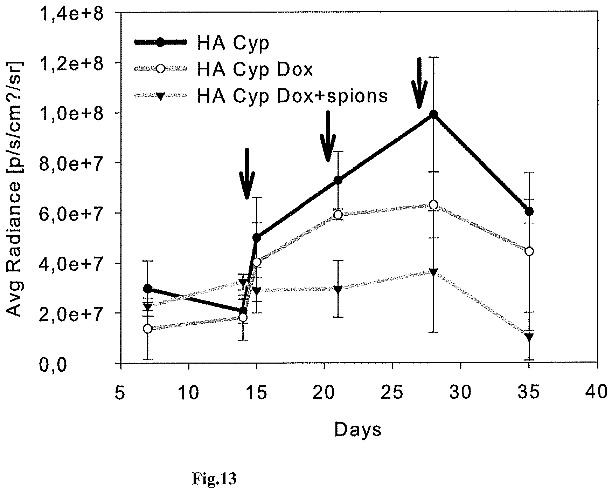

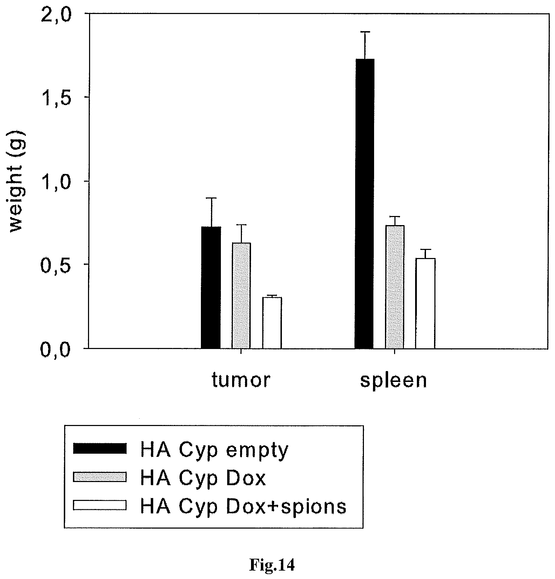

To 9 model mice Balb/c (in narcosis) were orthotopically administered 4T1 luc cells, (cells displayable with chemiluminescence -detection of luciferase activity after i.p. application of luciferin) and breast tumor was let grow for 14 days. 14^{th} day were mice divided into 3 groups (1 group = 3 animals). To every group was 14^{th}, 21^{th} and 28^{th} day intravenously applied 1 mg of selected conjugate in 100 µl phosphate buffer and then: to first group was applied HA-Cypate-C18:1 prepared in Example 15, to second group HA-Cypate-C18:1 loaded with doxorubicin, prepared in Example 23 and to third group HA-Cypate-C18:1 loaded with doxorubicin and spions, prepared in Example 24. The radiation was evaluated for 35 days *in vivo* (Fig. 13), then mice were sacrificed and the tumor and spleen weight in individual groups was compared (Fig. 14).

Fig. 13 shows that the tumor grew most in the group, to which only HA-Cypate was administered. Slower grow was detected in contrast in groups, to which HA-Cypate-C18:1+doxorubicin, and/or HA-Cypate-C18:1+doxorubicin+spion was applied.

Fig. 14 shows, that the first (not treated) group had the highest spleen and tumor weight. In given model, increased spleen indicates progression of disease (duPre et al., Experimental and Molecular Pathology 2007, 82, 12-24). The second (treated) and the third (treated) group had spleen significantly smaller and tumor smaller compared to the first group. The smallest tumor size was found in the third group. The carrier system can thus not only image but also cure the tumor and fills the function of theranostatic.

### Example 29: In vivo experiment -fluorescence of conjugate HA-Cypate-C18:1

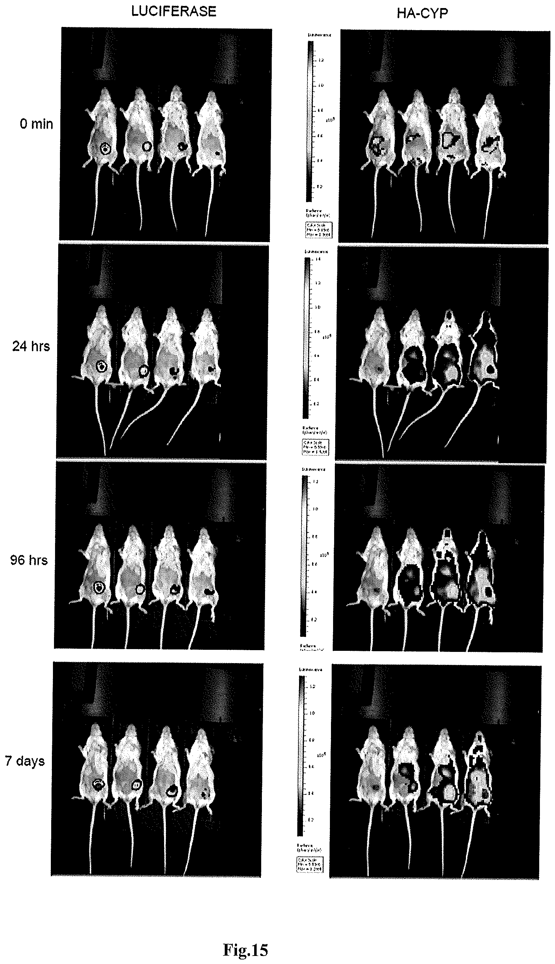

HA-Cypate-C18:1 (SS =10 %) prepared according to Example 15 was dissolved in the saline solution (c = 0; 0.625; 1.25; 2.5 mg of the conjugate in 100 µl solution) and sterilized by the filtration (0.22 µm). 100 µl of such prepared solutions was applied intraperitoneally to 4 model mice Balb/C (in narcosis) with the breast tumor (35^{th} day after orthotopic administration of 4T1 luc cells, displayable with chemiluminescence detection of luciferase activity after i.p. injection of luciferin) and observed its fluorescence *in vivo* for 7 days (Fig. 15).

Fig. 15 shows, that HA-Cypate-C18:1 is distributed after the i.p. administration into tumor and liver. The tumor is without any problem displayable *in vivo* at minimum for 7 days after the administration of the conjugate in all concentrations used. In left panel of the figure is the control image of the tumor with the luminescent imaging (detection of luciferase). The right panel of the figure shows 4 mice, to which the solution of the derivative was administered - from left to right grows the concentration: (0; 0.625; 1.25; 2.5 mg of the conjugate administered in 100 µl solution).

## Claims

1. A fluorescent conjugate of hyaluronic acid or a salt thereof of the general formula I, 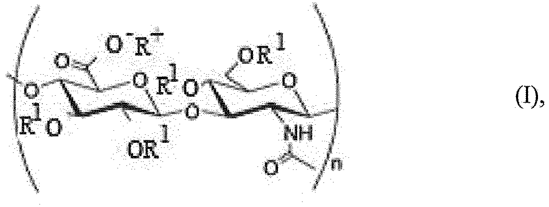
wherein R⁺ is H⁺ or physiologically acceptable salt selected from the group containing Na⁺, K⁺, Mg²⁺ or Ca²⁺,
R¹ is -H or a cypate residue of the formula II, where ∼ is in the place of covalent bond of cypate residue of the formula II 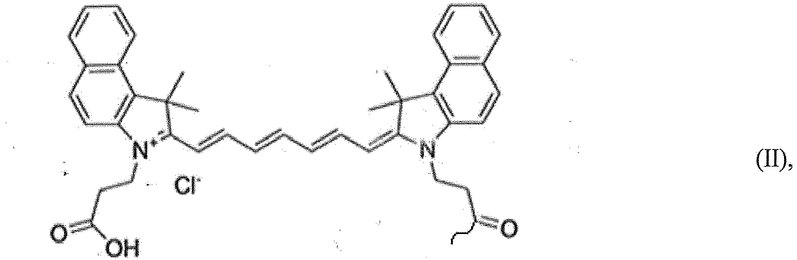
one R¹ being cypate residue of formula II in at least one repeated unit providing that if there is R¹ cypate residue of formula II in the unit, then the others R¹ in the unit are H,
and wherein n is an integer in the range 2 to 625.

2. The fluorescent conjugate of Claim 1, where the residue of cypate of the formula II is in the position 6 of the glucosamine part of the fluorescent conjugate of the hyaluronic acid or the salt thereof of the general formula I.

3. The fluorescent conjugate of Claim 1 or Claim 2, where the degree of substitution of the residue of cypate of the formula II in the conjugate of hyaluronic acid or the salt thereof of the general formula I is from 0.1 to 2%, preferably 1.0%.

4. The fluorescent conjugate of any one of Claims 1 to 3 that absorbs the light in the area of 570 nm to 790 nm and emits the light in the area of 680 nm to 850 nm, preferably at 850 nm.

5. A hydrophobized derivate of fluorescent conjugate of hyaluronic acid or the salt thereof of any one of Claims 1 to 3, wherein R⁺, R¹ and n are, as defined in Claim 1, applying at the same time, that in at least one repeated unit at least one R¹ is -C(=O)R², where R² is CₓH_{y} substituent, where x is an integer in the range of 5 to 17 and y is an integer 11 to 35, wherein it is a linear or branched, saturated or unsaturated C₆-C₁₈ aliphatic chain.

6. The hydrophobized derivate of Claim 5, wherein the degree of substitution of - C(=O)R² in the conjugate of hyaluronic acid or the salt thereof of the general formula I is from 3 to 70 %, preferably 5 to 12%.

7. The hydrophobized derivate of Claim 5 or Claim 6, that absorbs the light in the range of wavelengths 570 nm to 790 nm and emits the light at 680 to 850 nm, preferably 850 nm.

8. A method of preparation of the conjugate of any one of Claims 1 to 4, **characterized in, that** cypate I of the formula III 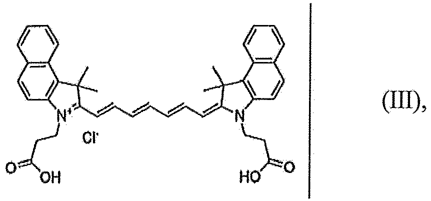 is activated with N,N'-carbonyl diimidazole (CDI) in the aprotic polar solvent selected from the group containing dimethyl sulfoxide, dimethyl formamide, formamide or acetonitrile, preferably dimethyl sulfoxide; resulting in a reactive intermediate mono-imidazolide of the formula IV 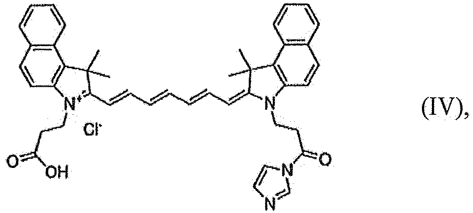 that reacts with hyaluronic acid or the salt thereof in the presence of the organic base, that is either generated *in situ* in the form of imidazole, or it is added to the reaction mixture, the added organic base being selected from the group containing 1,4-diazabicyclo[2.2.2]octane, *N,N,N',N'*-tetramethyl-1,6-hexanediamine, *N*-methyl morpholine, imidazole, triethylamine, or *N,N'*-diisopropyl ethylamine, preferably imidazole generated *in situ;* and polar aprotic solvent, as is defined above.

9. The method of Claim 8, **characterized in, that** the activation of cypate is performed at the temperature in the range 20 °C to 60 °C, preferably at 22 °C to 25 °C; for 10 minutes to 20 hours, preferably 0.5 to 2 hours

10. The method of Claim 8, **characterized in, that** the formation of the conjugate of hyaluronic acid or the salt thereof is performed at temperature 40 °C to 80 °C, preferably 40 °C to 60 °C, more preferably 60 °C; for 12 to 48 hours, preferably 24 hours.

11. The method of any one of Claims 8 to 10, **characterized in, that** the molar ratio of cypate I: hyaluronic acid or the salt thereof: N,N'-carbonyl diimidazole : the organic base is 0.5 : 1 : 0.5 : 0.5 to 3.5 in the reaction mixture, preferably the molar ratio is 0.5 : 1 : 0.5 : 1.

12. A method of a preparation of the hydrophobized derivate of fluorescent conjugate of any one of Claims 5 to 7, **characterized in, that** the activation of the fat acid of the general formula V is performed
R²COOH (V),
wherein R² is CₓH_{y}, whereas x is an integer in the range 5 to 17 and y is an integer 11 to 35 and CₓH_{y} is linear or the branched, saturated or unsaturated chain; using the substituted or unsubstituted benzoyl chloride of the general formula VI 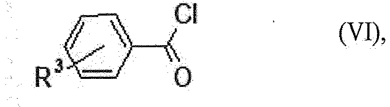
wherein R³ is one or more substituents selected from the group containing H, -NO₂, -COOH, halogenides, C₁-C₆ alkyl alkoxy, preferably H;
in the presence of the organic base selected from the group containing 1,4-diazabicyclo[2.2.2]octane, N,N,N',N'-tetramethyl-1,6-hexanediamine, N-methyl morpholine, triethylamine or N,N'-diisopropyl ethylamine, preferably triethylamine; and the polar solvent selected from the group containing isopropyl alcohol, tetrahydrofuran, preferably isopropyl alcohol
to form the reactive anhydride of the general formula VII 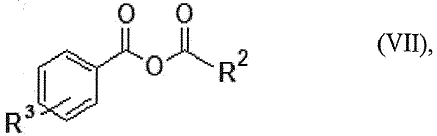 wherein
R² and R³ are, as is defined above,
that esterifies the fluorescent conjugate of hyaluronic acid or the salt thereof of the general formula (I), as defined in any one of Claims 1 to 3, in the presence of the organic base, preferably amine selected from the group containing 1,4-diazabi-cyclo[2.2.2]octane, N,N,N',N'-tetramethyl-1,6-hexanediamine, N-methyl morpholine, imidazole, triethylamin or N,N'-diisopropyl ethylamine, more preferably triethylamine; the mixture of water and the polar solvent miscible with water selected from the group containing isopropyl alcohol, dimethyl sulfoxide or tetrahydrofuran, preferably isopropyl alcohol.

13. The method of Claim 12, **characterized in, that** the activation of the fat acid of the general formula V is performed for 0.5 to 24 hours, at the temperature in the range 0 °C to 60 °C, preferably 0.5 hours at the temperature from 0 °C to 25 °C, and the esterification of the fluorescent conjugate of hyaluronic acid or the salt thereof is performed for 0.5 to 2 hours, preferably 2 hours, at the temperature in the range of 22 °C to 25 °C.

14. The method of Claim 12 or Claim 13 **characterized in, that**
the amount of the organic base corresponds to 2 to 6 molar equivalents, preferably 4 molar equivalents per the dimer of hyaluronic acid or the salt thereof;
the amount of the substituted or unsubstituted benzoyl chloride corresponds to 0.2 to 2.0 molar equivalents, preferably 0.6 molar equivalents per the dimer of hyaluronic acid or the salt thereof;
the amount of the fat acid corresponds to 0.2 to 2.0 molar equivalents, preferably 0.6 molar equivalents of hyaluronic acid or the salt thereof.

15. The method of any one of claims 12 to 14 **characterized in, that** the amount of water in the mixture water and the polar solvent miscible with water is 50 to 80 % v/v, preferably 50% v/v.

16. The fluorescent conjugate of any one of Claims 1 to 4 or the hydrophobized derivate thereof of any of claims 5 to 7 for use in medicinal applications for *in vivo* imaging of the conjugate distribution, preferably for *in vivo* imaging of organs or neoplasms.

17. The fluorescent conjugate or the hydrophobized derivate thereof of claim 16 for use in the intravenous, intraperitoneal or subcutaneous application.

18. The fluorescent conjugate or the hydrophobized derivate thereof of claim 16 for use in administration for *in vivo* imaging of non-palpable and/or palpable tumors.

19. The fluorescent conjugate or the hydrophobized derivate thereof of claim 18 for use in the intravenous, intraperitoneal administration.

20. A composition on the basis of aggregated the hydrophobized derivate of fluorescent conjugate of any one of Claims 5 to 7 **characterized in that** it contains an aggregate of the hydrophobized derivates of fluorescent conjugates and at least one or more nonpolar agents, preferably drugs and/or nanoparticles.

21. The composition of Claim 20 **characterized in that** the drug is a cytostatic, preferably doxorubicin or paclitaxel.

22. The composition of Claim 20 **characterized in that** the hydrophobized derivate of fluorescent conjugate has R¹ -C(=O)C₁₇H₃₃ and nanoparticles are superparamagnetic nanoparticles.

23. The composition of any one of Claims 20 to 22 **characterized in that** it contains 2 to 15 wt.%, preferably contains 2 to 6 wt.% of nonpolar compounds in respect to weight content of the hydrophobized derivate of fluorescent conjugate of hyaluronic acid or the salt thereof.

24. The composition of any one of Claims 20 to 23 for use in medicinal applications for *in vivo* imaging of neoplasms.

25. The composition of any one of Claims 20 to 23 for use in the treatment of neoplasms.

## Patentansprüche

1. Ein Hyaluronsäurefluoreszenzkonjugat oder ein Salz desselben, nach der allgemeinen Formel I, 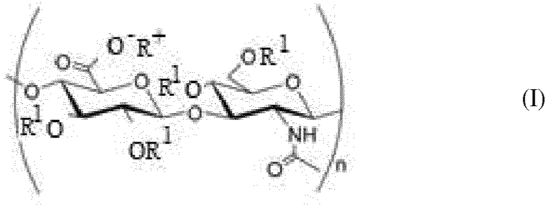
wobei R⁺ für H⁺ oder für ein physiologisch annehmbares Salz steht, das aus der Gruppe ausgewählt ist, die Na⁺, K⁺, Mg²⁺ oder Ca²⁺ umfasst,
R¹ für -H oder für einen Cypaterest nach der Formel II steht, wobei mit ∼ die kovalente Bindung des Cypaterest nach der Formel II bezeichnet ist 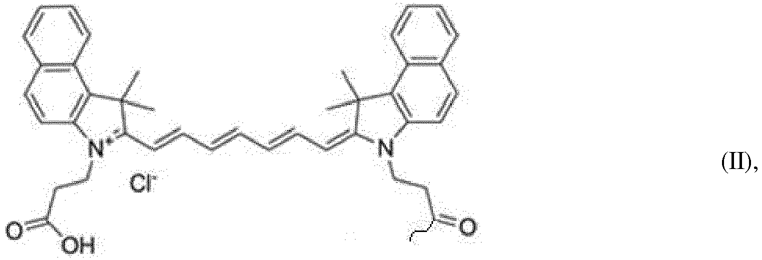
eine der R¹-Gruppen für den Cypaterest nach der Formel II in mindestens einer sich wiederholenden Einheit steht, unter Voraussetzung, dass wenn es innerhalb der Einheit eine von dem Cypaterest nach der Formel II gebildete R¹-Gruppe gibt, dann sind die anderen R¹-Gruppen innerhalb der Einheit H,
und wobei n eine im Bereich von 2 bis 625 liegende ganze Zahl ist.

2. Das Fluoreszenzkonjugat nach Anspruch 1, wobei der Cypaterest nach der Formel II sich in der Position 6 des Glukosaminteils des Hyaluronsäurefluoreszenzkonjugats oder eines Salzes desselben nach der allgemeinen Formel I befindet.

3. Das Fluoreszenzkonjugat nach Anspruch 1 oder Anspruch 2, wobei der Substitutionsgrad des Cypaterest nach der Formel II in dem Hyaluronsäurekonjugat oder einem Salz desselben nach der allgemeinen Formel I im Bereich von 0,1 bis 2 %, vorzugsweise 1,0 %, liegt.

4. Das Fluoreszenzkonjugat nach einem der Ansprüche 1 bis 3, das Licht mit Wellenlängen im Bereich von 570 nm bis 790 nm absorbiert und Licht mit Wellenlängen im Bereich von 680 nm bis 850 nm, vorzugsweise mit der Wellenlänge von 850 nm, emittiert.

5. Ein hydrophobisiertes Derivat des Hyaluronsäurefluoreszenzkonjugats oder eines Salzes desselben nach einem der Ansprüche 1 bis 3, wobei R⁺, R¹ und n im Anspruch 1 definiert sind und wobei es zugleich gilt, dass in mindestens einer sich wiederholenden Einheit die R¹-Gruppe für -C(=O)R² steht, wobei die R²-Gruppe für einen CₓH_{y}-Substituenten steht, wobei x eine ganze Zahl im Bereich von 5 bis 17 und y eine ganze Zahl im Bereich von 11 bis 35 ist, wobei die Gruppe eine C₆-C₁₈-aliphatische Kette ist, die linear oder verzweigt, sowie gesättigt oder ungesättigt ist.

6. Das hydrophobisierte Derivat nach Anspruch 5, wobei der Substitutionsgrad von -C(=O)R² in dem Hyaluronsäurekonjugat oder einem Salz desselben nach der allgemeinen Formel I im Bereich von 3 bis 70 %, vorzugsweise in dem Bereich von 5 bis 12 %, liegt.

7. Das hydrophobisierte Derivat nach Anspruch 5 oder Anspruch 6, das Licht mit Wellenlängen im Bereich von 570 nm bis 790 nm absorbiert und Licht mit Wellenlängen im Bereich von 680 nm bis 850 nm, vorzugsweise mit der Wellenlänge von 850 nm, emittiert.

8. Ein Verfahren zur Herstellung des Konjugats nach einem der Ansprüche 1 bis 4, **dadurch gekennzeichnet, dass** der Cypate I nach der Formel III 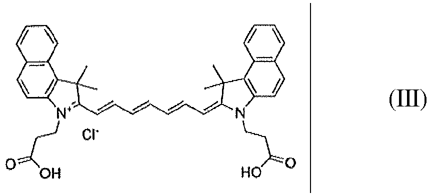 mit N,N'-Carbonyl-Diimidazole (CDI) in einem polaren aprotischen Lösungsmittel aktiviert wird, wobei das Lösungsmittel aus der Gruppe ausgewählt ist, die Dimethylsulfoxid, Dimethylformamid, Formamid oder Acetonitril, vorzugsweise Dimethylsulfoxid, umfasst, wobei die Aktivierung zur Bildung eines reaktiven intermediären Mono-Imidazolid nach der Formel IV führt, 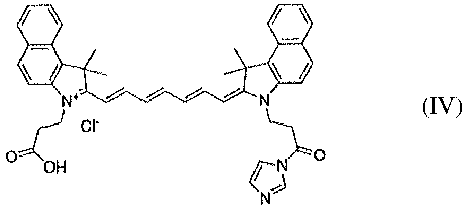 das mir Hyaluronsäure oder mit deren Salz in Gegenwart einer organischen Base reagiert, wobei die organische Base entweder *in situ* in der Form des Imidazols entsteht oder in dem Reaktionsgemisch zugegeben wird, wobei die derart zugegebene organische Base aus der Gruppe ausgewählt ist, die 1,4-Diazabizyklo-[2.2.2]Oktan, *N,N,N',N'-*Tetramethyl-1,6-Hexanediamin, *N*-Methyl-Morpholin, Imidazol, Triethylamin oder *N,N'-*Diisopropyl-Ethylamin, vorzugsweise das *in situ* entstehende Imidazol, umfasst, sowie in Gegenwart eines polaren aprotischen Lösungsmittels nach der obigen Definition.

9. Das Verfahren nach Anspruch 8, **dadurch gekennzeichnet, dass** die Aktivierung des Cypate 10 Minuten bis 20 Stunden lang, vorzugsweise 0,5 bis 2 Stunden lang, bei einer Temperatur im Bereich von 20 °C bis 60 °C, vorzugsweise im Bereich von 22 °C bis 25 °C, durchgeführt wird.

10. Das Verfahren nach Anspruch 8, **dadurch gekennzeichnet, dass** die Bildung des Hyaluronsäurekonjugats oder eines Salzes desselben, 12 bis 48 Stunden lang, vorzugsweise 24 Stunden lang, bei einer Temperatur im Bereich von 40 °C bis 80 °C, vorzugsweise im Bereich von 40 °C bis 60 °C, vorzugsweise bei der Temperatur von 60 °C durchgeführt wird.

11. Das Verfahren nach einem der Ansprüche 8 bis 10, **dadurch gekennzeichnet, dass** das molare Verhältnis Cypate I : Hyaluronsäure oder deren Salz : N,N'-CarbonylDiimidazol : die organische Base 0,5 : 1 : 0,5 : 0,5 bis 3.5 in dem Reaktionsgemisch beträgt, wobei vorzugsweise das molare Verhältnis 0,5 : 1 : 0,5 : 1 beträgt.

12. Ein Verfahren zur Herstellung des hydrophobisierten Fluoreszenzkonjugatderivats nach einem der Ansprüche 5 bis 7, **dadurch gekennzeichnet, dass** die Aktivierung der Fettsäure nach der allgemeinen Formel V durchgeführt wird,
R²COOH (V),
wobei R² für CₓH_{y} steht, wobei x eine ganze Zahl im Bereich von 5 bis 17 und y eine ganze Zahl im Bereich von 11 bis 35 ist und wobei CₓH_{y} eine aliphatische Kette ist, die linear oder verzweigt, sowie gesättigt oder ungesättigt ist, wobei die Aktivierung unter Verwendung des substituierten oder nicht substituierten Benzoylchlorids nach der allgemeinen Formel VI erfolgt, 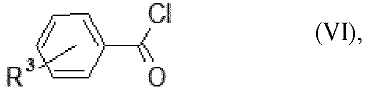
wobei R³ ein oder mehrere Substituenten sind, die aus der Gruppe ausgewählt sind, die H, -NO₂, -COOH, Halogenid, C₁-C₆-Alkylakoxy, vorzugsweise H, umfasst,
in Gegenwart einer organischen Base, die aus der 1,4-Diazabizyklo-[2.2.2]Oktan, N,N,N',N'-Tetramethyl-1,6-Hexanediamin, N-Methyl-Morpholin, Triethylamin oder N,N'-Diisopropyl-Ethylamin, vorzugsweise Triethylamin, umfassenden Gruppe ausgewählt ist, sowie in Gegenwart eines polaren Lösungsmittels, das aus der Isopropylalkohol und Tetrahydrofuran, vorzugsweise Isopropylalkohol, umfassenden Gruppe ausgewählt ist,
um den reaktiven Anhydrid nach der allgemeinen Formel VII zu bilden, 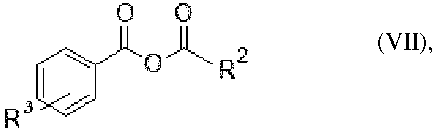 wobei
R² und R³ den obigen Definitionen entsprechen,
der die Esterifizierung des in einem der Ansprüche 1 bis 3 definierten Hyaluronsäurefluoreszenzkonjugats oder eines Salzes desselben nach der allgemeinen Formel (I) bewirkt, wobei die Esterifizierung in Gegenwart einer organischen Base, vorzugsweise Amins aus der 1,4-Diazabizyklo-[2.2.2]Oktan, N,N,N',N'-Tetramethyl-1,6-Hexanediamin, N-Methyl-Morpholin, Imidazol, Triethylamin oder N,N'-DiisopropylEthylamin, vorzugsweise Triethylamin, umfassenden Gruppe ausgewählten, sowie in Gegenwart eines aus Wasser und einem wassermischbaren polaren Lösungsmittel bestehenden Gemisches stattfindet, wobei das Lösungsmittel aus der Gruppe ausgewählt ist, die Isopropylalkohol, Dimethylsulfoxid oder Tetrahydrofuran, vorzugsweise Isopropylalkohol, umfasst.

13. Das Verfahren nach Anspruch 12, **dadurch gekennzeichnet, dass** die Aktivierung der Fettsäure nach der allgemeinen Formel V im Zeitraum von 0,5 bis 24 Stunden bei einer Temperatur im Bereich von 0 °C bis 60 °C, vorzugsweise 0,5 Stunden lang bei einer Temperatur im Bereich von 0 °C bis 25 °C, durchgeführt wird und die Esterifizierung des Hyaluronsäurefluoreszenzkonjugats oder eines Salzes desselben im Zeitraum von 0,5 bis 2 Stunden, vorzugsweise 2 Stunden lang, bei einer Temperatur im Bereich von 22 °C bis 25 °C durchgeführt wird.

14. Das Verfahren nach Anspruch 12 oder Anspruch 13, **dadurch gekennzeichnet, dass** die Menge der organischen Base 2 bis 6 molaren Äquivalenten, vorzugsweise 4 molaren Äquivalenten, bezogen auf das Hyaluronsäuredimer oder einem Salz derselben, entspricht;
die Menge des substituierten oder nicht substituierten Benzoylchlorids 0,2 eines molaren Äquivalentes bis 2,0 molaren Äquivalenten, vorzugsweise 0,6 eines molaren Äquivalentes, bezogen auf das Hyaluronsäuredimer oder einem Salz derselben, entspricht;
die Menge der Fettsäure 0,2 eines molaren Äquivalentes bis 2,0 molaren Äquivalenten, vorzugsweise 0,6 eines molaren Äquivalentes, bezogen auf die Hyaluronsäure oder auf ein Salz derselben, entspricht.

15. Das Verfahren nach einem der Ansprüche 12 bis 14, **dadurch gekennzeichnet, dass** der Volumenanteil von Wasser in dem aus Wasser und einem aus Wasser und einem wassermischbaren polaren Lösungsmittel bestehenden Gemisch 50 bis 80 % v/v, vorzugsweise 50 % v/v beträgt.

16. Das Fluoreszenzkonjugat nach einem der Ansprüche 1 bis 4 oder dessen hydrophobisiertes Derivat nach einem der Ansprüche 5 bis 7, zur Anwendung in den medizinischen Applikationen bei der In vivo Bildgebung der Konjugatsverteilung, vorzugsweise bei der In vivo Bildgebung von Organen oder Neoplasmen.

17. Das Fluoreszenzkonjugat oder dessen hydrophobisiertes Derivat nach Anspruch 16, zur Anwendung in den intravenösen, intraperitonealen oder subkutanen Verabreichungen.

18. Das Fluoreszenzkonjugat oder dessen hydrophobisiertes Derivat nach Anspruch 16, zur Anwendung in den Verabreichungen bei der *In vivo* Bildgebung von untastbaren und/oder tastbaren Tumoren.

19. Das Fluoreszenzkonjugat oder dessen hydrophobisiertes Derivat nach Anspruch 18, zur Anwendung in den intravenösen, intraperitonealen Verabreichungen.

20. Eine Zusammensetzung auf der Basis des aggregierten hydrophobisierten Fluoreszenzkonjugatderivats nach einem der Ansprüche 5 bis 7, **dadurch gekennzeichnet, dass** sie ein Aggregat der hydrophobisierten Fluoreszenzkonjugatderivate und mindestens ein nicht-polares Mittel, vorzugsweise Arzneimittel und/oder Nanopartikel, umfasst.

21. Die Zusammensetzung nach Anspruch 20, **dadurch gekennzeichnet, dass** das Arzneimittel ein Zytostatikum, vorzugsweise Doxorubicin oder Paclitaxel, ist.

22. Die Zusammensetzung nach Anspruch 20, **dadurch gekennzeichnet, dass** das hydrophobisierte Fluoreszenzkonjugatderivat die Gruppe R¹ -C(=O)C₁₇H₃₃ umfasst und dass die Nanopartikel superparamagnetische Nanopartikel sind.

23. Die Zusammensetzung nach einem der Ansprüche 20 bis 22, **dadurch gekennzeichnet, dass** sie nicht-polare Verbindungen in der Menge von 2 bis 15 Gew.-%, vorzugsweise 2 bis 6 Gew.-%, bezogen auf den Gewichtsanteil des hydrophobisierten Hyaluronsäurefluoreszenzkonjugatderivats oder einem Salz desselben, umfasst.

24. Die Zusammensetzung nach einem der Ansprüche 20 bis 23, zur Anwendung in den medizinischen Applikationen bei der *In vivo* Bildgebung von Neoplasmen.

25. Die Zusammensetzung nach einem der Ansprüche 20 bis 23, zur Anwendung bei der Behandlung von Neoplasmen.

## Revendications

1. Conjugué fluorescent de l'acide hyaluronique ou son sel de formule générale I, 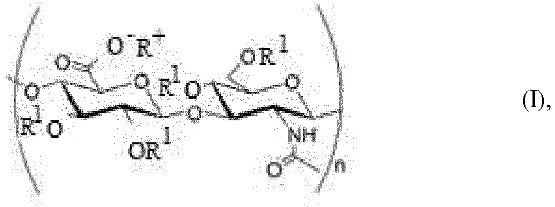
où R⁺ est H⁺ ou un sel physiologiquement acceptable choisi dans le groupe comprenant Na⁺, K⁺, Mg²⁺ ou Ca²⁺,
R¹ est -H ou le résidu de cypate de formule II, où ∼ est le site de liaison covalente du résidu de cypate de formule II, 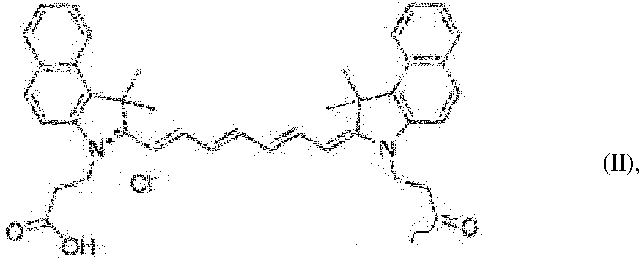
dans au moins une unité de répétition un R¹ étant le résidu de cypate de formule II, et si dans l'unité un R¹ est le résidu de cypate, les autres R¹ dans l'unité sont H,
et où n est un nombre entier compris entre 2 et 625.

2. Conjugué fluorescent selon la revendication 1, où le résidu de cypate de formule II est en position 6 de la partie glucosamine du conjugué fluorescent de l'acide hyaluronique ou de son sel de formule générale I.

3. Conjugué fluorescent selon la revendication 1 ou la revendication 2, où le degré de substitution du résidu de cypate de formule II dans le conjugué de l'acide hyaluronique ou son sel de formule générale I est compris entre 0,1 et 2 %, de préférence 1,0 %.

4. Conjugué fluorescent selon l'une quelconque des revendications 1 - 3, qui absorbe la lumière dans le domaine entre 570 nm et 790 nm et émet la lumière dans le domaine entre 680 nm et 850 nm, de préférence à 850 nm.

5. Dérivé hydrophobisé du conjugué fluorescent de l'acide hyaluronique ou son sel selon l'une quelconque des revendications 1 - 3, où R⁺, R¹ et n sont tel que défini dans la revendication 1, dans au moins une unité de répétition au moins un R¹ étant -C(=O)R², où R² est CₓH_{y} substituant, où x est un nombre entier compris entre 5 et 17 et y est un nombre entier compris entre 11 et 35, étant donné qu'il s'agit d'une chaîne aliphatique C₆-C₁₈, linéaire ou ramifiée, saturée ou insaturée.

6. Dérivé hydrophobisé selon la revendication 5, où le degré de substitution -C(=O)R² dans le conjugué de l'acide hyaluronique ou son sel de formule générale I est compris entre 3 et 70 %, de préférence entre 5 et 12 %.

7. Dérivé hydrophobisé selon la revendication 5 ou la revendication 6, qui absorbe la lumière dans le domaine des longueurs d'onde entre 570 nm et 790 nm et émet la lumière entre 680 et 850 nm, de préférence à 850 nm.

8. Procédé de préparation du conjugué selon l'une quelconque des revendications 1 - 4, **caractérisé en ce que** le cypate de formule III 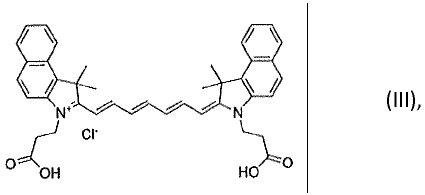 est activé à l'aide du N,N'-carbonyldiimidazole (CDI) dans un solvant polaire aprotique choisi dans le groupe comprenant le diméthylsulfoxyde, le diméthylformamide, le formamide ou l'acétonitrile, de préférence le diméthylsulfoxyde, en formant un intermédiaire réactif mono-imidazolide de formule IV 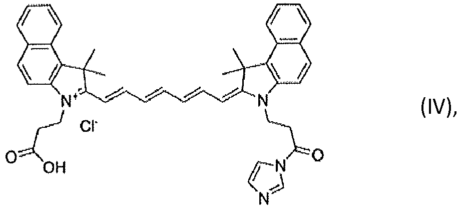 qui réagit avec l'acide hyaluronique ou son sel en présence d'une base organique, celle-ci étant soit générée *in situ* sous forme de l'imidazole, ou ajoutée au mélange réactionnel, la base organique ajoutée étant choisie dans le groupe comprenant le (1,4-diazabicyclo[2.2.2]octane), la *N,N,N',N*'-tétraméthyle-1,6-hexandiamine, la *N-*méthylmorpholine, l'imidazole, la triéthylamine, ou la *N,N'*-diisopropyléthylamine, de préférence l'imidazole générée *in situ ;* et d'un solvant polaire aprotique, tel que défini ci-dessus.

9. Procédé selon la revendication 8, **caractérisé en ce que** l'activation du cypate est effectuée à une température comprise entre 20°C et 60 °C, de préférence entre 22°C et 25 °C, pendant une durée allant de 10 minutes à 20 heures, de préférence de 0,5 à 2 heures.

10. Procédé selon la revendication 8, **caractérisé en ce que** la formation du conjugué de l'acide hyaluronique ou de don sel est effectuée à une température comprise entre 40°C et 80°C, de préférence entre 40°C et 60°C, encore mieux à 60°C, pendant une durée allant de 12 à 48 heures, de préférence 24 heures.

11. Procédé selon l'une quelconque des revendications 8 - 10, **caractérisé en ce que** le rapport molaire cypate I : acide hyaluronique ou son sel : N,N'-carbonyldiimidazole : base organique est 0,5 : 1 : 0,5 : 0,5 - 3,5 dans le mélange réactionnel, de préférence le rapport molaire est 0,5 : 1: 0,5 : 1.

12. Procédé de préparation du dérivé hydrophobisé du conjugué fluorescent selon l'une quelconque des revendications 5 - 7, **caractérisé en ce qu'**on effectue l'activation de l'acide gras de formule générale V
R²COOH (V),
où R² est CₓH_{y}, x étant le nombre entier compris entre 5 et 17 et y étant le nombre entier compris entre 11 et 35 et CₓH_{y} est une chaîne linéaire ou ramifiée, saturée ou insaturée, à l'aide d'une chlorure de benzoyl substituée ou non substituée de formule générale VI 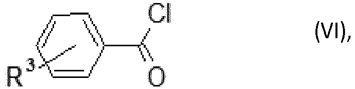
où R³ représente un ou plusieurs substituants choisis dans le groupe comprenant H, -NO₂, -COOH, les halogénures, C₁-C₆ alkylalcoxy, de préférence H,
en présence d'une base organique choisie dans le groupe comprenant le 1,4-diazabicyclo[2.2.2]octane, la *N,N,N',N*'-tétraméthyle-1,6-hexandiamine, la *N-*méthylmorpholine, la triéthylamine ou la *N,N'*-diisopropyléthylamine, de préférence la triéthylamine ; et d'un solvant polaire choisi dans le groupe comprenant l'alcool isopropylique, le tétrahydrofurane, de préférence l'alcool isopropylique
en formant un anhydride réactif de formule générale VII 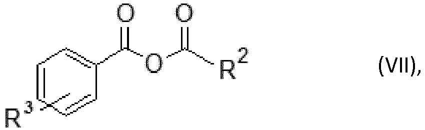 où
R² et R³ sont tels que défini ci-dessus,
qui ésterifie le conjugué fluorescent de l'acide hyaluronique ou son sel de formule générale (I), tel que défini dans l'une quelconque des revendications 1 - 3, en présence d'une base organique, de préférence d'une amine choisie dans le groupe comprenant le 1,4-diazabicyclo[2.2.2]octane, la *N,N,N',N*'-tétraméthyle-1,6-hexandiamine, la *N-*méthylmorpholine, l'imidazole, la triéthylamine ou la *N,N'*-diisopropyléthylamine, de préférence la triéthylamine ; d'un mélange d'eau et d'un solvant polaire miscible dans l'eau choisi dans le groupe comprenant l'alcool isopropylique, le diméthylsulfoxyde ou le tétrahydrofurane, de préférence l'alcool isopropylique.

13. Procédé selon la revendication 12, **caractérisé en ce** l'activation de l'acide gras de formule générale V est effectuée pendant une durée comprise entre 0,5 et 24 heures, à une température comprise entre 0°C et 60°C, de préférence 0,5 heures, à une température comprise entre 0°C et 25°C, et l'estérification du conjugué fluorescent de l'acide hyaluronique ou de son sel est effectuée pendant une durée comprise entre 0,5 et 2 heures, de préférence 2 heures, à une température comprise entre 22°C et 25°C.

14. Procédé selon la revendication 12 ou la revendication 13, **caractérisé en ce que**
la quantité de base organique correspond à 2 - 6 équivalents molaires, de préférence 4 équivalents molaires, par dimère de l'acide hyaluronique ou de son sel,
la quantité de chlorure de benzoyle substituée ou non substituée correspond à 0,2 - 2,0 équivalents molaires, de préférence 0,6 équivalents molaires, par dimère de l'acide hyaluronique ou de son sel,
la quantité d'acide gras correspond à 0,2 - 2,0 équivalents molaires, de préférence à 0,6 équivalents molaires, par dimère de l'acide hyaluronique ou de son sel.

15. Procédé selon l'une quelconque des revendications 12 - 14, **caractérisé en ce que** la quantité d'eau dans le mélange d'eau et de solvant polaire miscible dans l'eau est comprise entre 50 et 80 % vol./vol., de préférence 50 % vol./vol.

16. Conjugué fluorescent selon l'une quelconque des revendications 1 - 4 ou son dérivé hydrophobisé selon l'une quelconque des revendications 5 - 7 pour l'utilisation dans des applications médicales servant à l'imagerie *in vivo* de la distribution du conjugué, de préférence à l'imagerie *in vivo* des organes ou des néoplasmes.

17. Conjugué fluorescent ou son dérivé hydrophobisé selon la revendication 16 pour l'utilisation dans une administration intraveineuse, intraperitoneale ou sous-cutanée.

18. Conjugué fluorescent ou son dérivé hydrophobisé selon la revendication 16 pour l'utilisation dans une administration servant à l'imagerie *in vivo* des tumeurs non palpables et/ou palpables.

19. Conjugué fluorescent ou son dérivé hydrophobisé selon la revendication 18 pour l'utilisation dans une administration intraveineuse, intraperitoneale.

20. Composition sur la base du dérivé hydrophobisé du conjugué fluorescent agrégé selon l'une quelconque des revendications 5 - 7, **caractérisée en ce qu'**elle comprend un agrégat des dérivés hydrophobisés des conjugués fluorescents et au moins une ou plusieurs substances non polaires, de préférence médicaments et/ou nanoparticules.

21. Composition selon la revendication 20, **caractérisée en ce que** le médicament est un cytostatique, de préférence la doxorubicine ou le paclitaxel.

22. Composition selon la revendication 20, **caractérisée en ce que** le dérivé hydrophobisé du conjugué fluorescent a R¹-C(=O)C₁₇H₃₃ et les nanoparticules sont des nanoparticules superparamagnétiques.

23. Composition selon l'une quelconque des revendications 20 - 22, **caractérisée en ce qu'**elle comprend entre 2 et 15 % en poids, elle comprend de préférence entre 2 et 6 % en poids de substances non polaires par rapport à la teneur pondérale du dérivé hydrophobisé du conjugué fuorescent de l'acide hyaluronique ou de son sel.

24. Composition selon l'une quelconque des revendications 20 - 23 pour l'utilisation dans des applications médicales servant à l'imagerie *in vivo* des néoplasmes.

25. Composition selon l'une quelconque des revendications 20 - 23 pour l'utilisation dans le traitement des néoplasmes.
